# EUROPEAN PATENT SPECIFICATION

(11) **EP 2 530 166 B1**
(45) Date of publication and mention of the grant of the patent: **16.07.2014**
(21) Application number: 12170098.3
(22) Date of filing: 30.05.2012
(51) Int. Cl.: C12Q 1/68

(54) **Method for detecting a plurality of nucleotide polymorphisms at a single wavelength using a plurality of oligonucleotides modified with fluorescent dye having the same or close detection wavelength**
Verfahren zum Nachweis mehrerer Nukleotid-Polymorphismen auf einer einzelnen Wellenlänge mit mehreren mit fluoreszierendem Farbstoff modifizierten Oligonukleotiden mit derselben oder einer nahen Nachweiswellenlänge
Procédé pour détecter plusieurs polymorphismes nucléotidiques à une seule longueur d'onde au moyen de plusieurs oligonucléotides modifiés avec un colorant fluorescent ayant une longueur d'onde de détection identique ou proche

(30) Priority: 31.05.2011 JP 2011122900
(43) Date of publication of application: 05.12.2012
(73) Proprietor: ARKRAY, Inc., Kyoto-shi, Kyoto 601-8045 (JP)
(72) Inventor: Iguchi, Aki, Kyoto-shi, Kyoto 602-0008 (JP)
(74) Representative: Jones, Elizabeth Louise

(56) References cited:
- WITTWER C T ET AL: "Real-time multiplex PCR assays", METHODS : A COMPANION TO METHODS IN ENZYMOLOGY, ACADEMIC PRESS INC., NEW YORK, NY, US, vol. 25, no. 4, 1 December 2001 (2001-12-01), pages 430-442, XP002265718, ISSN: 1046-2023, DOI: 10.1006/METH.2001.1265
- MILLWARD H ET AL: "Homogeneous Amplification and Mutation Scanning of the p53 Gene Using Fluorescent Melting Curves", CLINICAL CHEMISTRY, AMERICAN ASSOCIATION FOR CLINICAL CHEMISTRY, WASHINGTON, DC, vol. 48, no. 8, 1 January 2002 (2002-01-01), pages 1321-1328, XP002317456, ISSN: 0009-9147

### SEQUENCE LISTING

<110> Arkray Inc.
<120> METHOD FOR DETECTING A PLURALITY OF NUCLEOTIDE POLYMORPHISMS AT A SINGLE WAVELENGTH USING A PLURALITY OF OLIGONUCLEOTIDES MODIFIED WITH FLUORESCENT DYE HAVING THE SAME OR CLOSE DETECTION WAVELENGTH
<130> 42.13.113049
<150> JP2011-122900
   <151> 2011-05-31
<160> 46
<170> PatentIn version 3.3
<210> 1
   <211> 50
   <212> DNA
   <213> Artificial Sequence
<220>
   <223> EGFR-e20-ins-WT-F
<400> 1
   gaagcctacg tgatggccag cgtggacaac ccccacgtgt gccgcctgct 50
<210> 2
   <211> 59
   <212> DNA
   <213> Artificial Sequence
<220>
   <223> EGFR-e20-ins9-F
<400> 2
   gaagcctacg tgatggccag cgtggccagc gtggacaacc cccacgtgtg ccgcctgct 59
<210> 3
   <211> 53
   <212> DNA
   <213> Artificial Sequence
<220>
   <223> EGFR-e20-insCAC-F
<400> 3
   gaagcctacg tgatggccag cgtggacaac ccccaccacg tgtgccgcct gct 53
<210> 4
   <211> 56
   <212> DNA
   <213> Artificial Sequence
<220>
   <223> EGFR-e20-insTGCGTG-F
<400> 4
   gaagcctacg tgatggccag cgtgtgcgtg gacaaccccc acgtgtgccg cctgct 56
<210> 5
   <211> 56
   <212> ' DNA
   <213> Artificial Sequence
<220>
   <223> EGFR-e20-insAACCCC-F
<400> 5
   gaagcctacg tgatggccag cgtggacaac cccaaccccc acgtgtgccg cctgct 56
<210> 6
   <211> 56
   <212> DNA
   <213> Artificial Sequence
<220>
   <223> EGFR-e20-insCCCCAC-F
<400> 6
   gaagcctacg tgatggccag cgtggacaac ccccaccccc acgtgtgccg cctgct 56
<210> 7
   <211> 59
   <212> DNA
   <213> Artificial Sequence
<220>
   <223> EGFR-e20-insAACCCCCAC-F
<400> 7
   gaagcctacg tgatggccag cgtggacaac ccccacaacc cccacgtgtg ccgcctgct 59
<210> 8
   <211> 56
   <212> DNA
   <213> Artificial Sequence
<220>
   <223> EGFR-e20-insCACGTG-F
<400> 8
   gaagcctacg tgatggccag cgtggacaac ccccacgtgc acgtgtgccg cctgct 56
<210> 9
   <211> 17
   <212> DNA
   <213> Artificial Sequence
<220>
   <223> 3PB-EGFR-insWT-R5
<400> 9
   tgtccacgct ggccatc 17
<210> 10
   <211> 19
   <212> DNA
   <213> Artificial Sequence
<220>
   <223> 3PB-EGFR-insWT-F4
<400> 10
   ggacaacccc cacgtgtgc 19
<210> 11
   <211> 20
   <212> DNA
   <213> Artificial Sequence
<220>
   <223> 3FL-EGFR-ins7-F1
<400> 11
   acaaccccca caacccccac 20
<210> 12
   <211> 50
   <212> DNA
   <213> Artificial Sequence
<220>
   <223> EGFR-e20-ins-WT-R1
<400> 12
   agcaggcggc acacgtgggg gttgtccacg ctggccatca cgtaggcttc 50
<210> 13
   <211> 59
   <212> DNA
   <213> Artificial Sequence
<220>
   <223> EGFR-e20-ins9-R2
<400> 13
   agcaggcggc acacgtgggg gttgtccacg ctggccacgc tggccatcac gtaggcttc 59
<210> 14
   <211> 53
   <212> DNA
   <213> Artificial Sequence
<220>
   <223> EGFR-e20-insCAC-R3
<400> 14
   agcaggcggc acacgtggtg ggggttgtcc acgctggcca tcacgtaggc ttc 53
<210> 15
   <211> 56
   <212> DNA
   <213> Artificial Sequence
<220>
   <223> EGFR-e20-insTGCGTG-R5
<400> 15
   agcaggcggc acacgtgggg gttgtccacg cacacgctgg ccatcacgta ggcttc 56
<210> 16
   <211> 56
   <212> DNA
   <213> Artificial Sequence
<220>
   <223> EGFR-e20-insAACCCC-R6
<400> 16
   agcaggcggc acacgtgggg gttggggttg tccacgctgg ccatcacgta ggcttc 56
<210> 17
   <211> 56
   <212> DNA
   <213> Artificial Sequence
<220>
   <223> EGFR-e20-insCCCCAC-R7
<400> 17
   agcaggcggc acacgtgggg gtgggggttg tccacgctgg ccatcacgta ggcttc 56
<210> 18
   <211> 59
   <212> DNA
   <213> Artificial Sequence
<220>
   <223> EGFR-e20-insAACCCCCAC-R8
<400> 18.
   agcaggcggc acacgtgggg gttgtggggg ttgtccacgc tggccatcac gtaggcttc 59
<210> 19
   <211> 56
   <212> DNA
   <213> Artificial Sequence
<220>
   <223> EGFR-e20-insCACGTG-R9
<400> 19
   agcaggcggc acacgtgcac gtgggggttg tccacgctgg ccatcacgta ggcttc 56
<210> 20
   <211> 17
   <212> DNA
   <213> Artificial Sequence
<220>
   <223> 3PB-858-G-PM
<400> 20
   ttggcccgcc caaaatc 17
<210> 21
   <211> 21
   <212> DNA
   <213> Artificial Sequence
<220>
   <223> 3PB-3A4-G-PM
<400> 21
   taaatcgccg ctctcctgcc c 21
<210> 22
   <211> 50
   <212> DNA
   <213> Artificial Sequence
<220>
   <223> 858-mm
<400> 22
   caagatcaca gattttgggc tggccaaact gctgggtgcg gaagagaaag 50
<210> 23
   <211> 50
   <212> DNA
   <213> Artificial Sequence
<220>
   <223> 858-PM
<400> 23
   caagatcaca gattttgggc gggccaaact gctgggtgcg gaagagaaag 50
<210> 24
   <211> 40
   <212> DNA
   <213> Artificial Sequence
<220>
   <223> 3A4-mm
<400> 24
   agccatagag acaagggcaa gagagaggcg atttaataga 40
<210> 25
   <211> 40
   <212> DNA
   <213> Artificial Sequence
<220>
   <223> 3A4-PM
<400> 25
   agccatagag acaagggcag gagagaggcg atttaataga 40
<210> 26
   <211> 17
   <212> DNA
   <213> Artificial Sequence
<220>
   <223> 3T-719-G-PM
<400> 26
   gcaccggagc ccagcac 17
<210> 27
   <211> 17
   <212> DNA
   <213> Artificial Sequence
<220>
   <223> 3T-858-G-PM
<400> 27
   ttggcccgcc caaaatc 17
<210> 28
   <211> 22
   <212> DNA
   <213> Artificial Sequence
<220>
   <223> 3T-790-T-PM
<400> 28
   tgagctgcat gatgaggtgc ac 22
<210> 29
   <211> 49
   <212> DNA
   <213> Artificial Sequence
<220>
   <223> 719-G
<400> 29
   gaattcaaaa agatcaaagt gctgggctcc ggtgcgttcg gcacggtgta 50
<210> 30
   <211> 50
   <212> DNA
   <213> Artificial Sequence
<220>
   <223> 719-C
<400> 30
   gaattcaaaa agatcaaagt gctggcctcc ggtgcgttcg gcacggtgta 50
<210> 31
   <211> 50
   <212> DNA
   <213> Artificial Sequence
<220>
   <223> 858-G
<400> 31
   caagatcaca gattttgggc gggccaaact gctgggtgcg gaagagaaag 50
<210> 32
   <211> 50
   <212> DNA
   <213> Artificial Sequence
<220>
   <223> 858-T
<400> 32
   caagatcaca gattttgggc tggccaaact gctgggtgcg gaagagaaag 50
<210> 33
   <211> 50
   <212> DNA
   <213> Artificial Sequence
<220>
   <223> 790-T
<400> 33
   cctcacctcc accgtgcagc tcatcatgca gctcatgccc ttcggctgcc 50
<210> 34
   <211> 50
   <212> DNA
   <213> Artificial Sequence
<220>
   <223> 790-C
<400> 34
   cctcacctcc accgtgcagc tcatcacgca gctcatgccc ttcggctgcc 50
<210> 35
   <211> 16
   <212> DNA
   <213> Artificial Sequence
<220>
   <223> 5PB-V12M-A-PM
<400> 35
   ccttgtgaca ttggga 16
<210> 36
   <211> 19
   <212> DNA
   <213> Artificial Sequence
<220>
   <223> 3PB-UGT-T-PM
<400> 36
   gagacagagc attttacac 19
<210> 37
   <211> 24
   <212> DNA
   <213> Artificial Sequence
<220>
   <223> 5FL-Q126X-T-PM
<400> 37
   cccacttata cttacttata ccac 24
<210> 38
   <211> 16
   <212> DNA
   <213> Artificial Sequence
<220>
   <223> 3FL-NAT-C-PM
<400> 38
   tgccgtcagt ggtcac 16
<210> 39
   <211> 50
   <212> DNA
   <213> Artificial Sequence
<220>
   <223> V12M-PM
<400> 39
   cagtaatgtc gaagttttta tcccaatgtc acaaggaaac accaatggct 50
<210> 40
   <211> 50
   <212> DNA
   <213> Artificial Sequence
<220>
   <223> V12M-mm
<400> 40
   cagtaatgtc gaagttttta tcccagtgtc acaaggaaac accaatggct 50
<210> 41
   <211> 40
   <212> DNA
   <213> Artificial Sequence
<220>
   <223> UGT-PM
<400> 41
   ttcaaggtgt aaaatgctct gtctctgatg tacaacgagg 40
<210> 42
   <211> 40
   <212> DNA
   <213> Artificial Sequence
<220>
   <223> UGT-mm
<400> 42
   ttcaaggtgt aaaatgctcc gtctctgatg tacaacgagg 40
<210> 43
   <211> 50
   <212> DNA
   <213> Artificial Sequence
<220>
   <223> Q126X-PM
<400> 43
   caaatgtaat tcaggttacg tggtataagt aagtattagt gggtttgcat 50
<210> 44
   <211> 50
   <212> DNA
   <213> Artificial Sequence
<220>
   <223> Q126X-mm
<400> 44
   caaatgtaat tcaggttacg tggtacaagt aagtattagt gggtttgcat 50
<210> 45
   <211> 40
   <212> DNA
   <213> Artificial Sequence
<220>
   <223> NAT-PM
<400> 45
   ccttctcctg caggtgacca ctgacggcag gaattacatt 40
<210> 46
   <211> 40
   <212> DNA
   <213> Artificial Sequence
<220>
   <223> NAT-mm
<400> 46
   ccttctcctg caggtgacca ttgacggcag gaattacatt 40

## Description

### TECHNICAL FIELD

The present invention relates to a method for detecting a plurality of nucleotide polymorphisms at a single wavelength using a plurality of oligonucleotides each modified with a fluorescent dye wherein the dyes are the same or similar to each other in terms of detection wavelength.

### BACKGROUND ART

There are many methods for analyzing a mutation(s) of a target nucleic acid using a fluorescent dye-labeled probe.

JP2002-355084A describes a mutation analysis method based on the results of melting curve analysis performed using a fluorescent dye-labeled nucleic acid probe after amplification of a region containing a mutation by PCR. In JP2002-355084A, as claim 13 states that "detection is performed at at least the same number of wavelengths as the number of kinds of novel nucleic acid probes for detection of nucleic acids", when a plurality of probes are used, the probes used are each labeled with a fluorescent dye having different wavelength.

JP2006-166808A describes a method for detecting a genetic mutation using a solid-phased probe, in which, as claim 8 states that "a plurality of kinds of fluorescent substances emitting fluorescent light with different wavelengths are bound", when a plurality of probes is used, the probes used are each labeled with a fluorescent dye having a different wavelength. Wittwer et al. (Methods: A Companion to Methods in Enzymology, Academic Press Inc., New York, NY, US, Vol. 25, No. 4, 2001, p430-442) describes identification of amplification products by their different fluorescence spectra and melting characteristics. Millward et al. (Clinical Chemistry, Vol. 48 No.8, 2002, p1321-1328) describes the use of overlapping fluorescein-labelled oligonucleotides in melting temperature analysis for mutation detection.

As mentioned above, in JP2002-355084A and JP2006-166808A, when using a plurality of probes, the probes are each labeled with a fluorescent dye having a different wavelength.

In JP2002-355084A and JP2006-166808A, it is stated that detection requires different fluorescent wavelengths in numbers equal to or more than the number of the probes (namely, the number of mutations desired to be detected). However, when there is a limit to the kinds of fluorescent wavelengths that can be detected by a detection apparatus or when there is a limit to the kinds of fluorescent molecules that can be modified on a probe, the number of detectable mutations is also limited. Additionally, in such a case, it is necessary to detect different genetic mutations several times using different reagents, which requires time and expense.

### SUMMARY OF THE INVENTION

It is an object of the present invention to provide a method for detecting many different nucleotide polymorphisms at one time, even when there is a limit to the kinds of fluorescent wavelengths detectable by a detection apparatus or even when there is a limit to the kinds of fluorescent molecules that can be modified on a probe.

The present inventor has found that a plurality of nucleotide polymorphisms can be simultaneously detected at a single detection wavelength by adding, to a single reaction system, a plurality of probes labeled with a fluorescent dye that are the same or close (similar) to each other in terms of detection wavelength and designed such that their Tm values are close (similar) to each other, thereby completing the present invention.

Specifically, the present invention is as follows.
(1) A method for simultaneously detecting a plurality ofnucleotide polymorphisms by melting curve analysis, comprising detecting the plurality of nucleotide polymorphisms at a single wavelength by using a plurality of oligonucleotides labeled with a fluorescent dye, each of which hybridizes to a region of a target sequence containing each (i.e. one or at least one) of the plurality of nucleotide polymorphisms, wherein
   the fluorescent dyes labeling the plurality of oligonucleotides are the same or close (similar) to each other in terms of detection wavelength;
   the plurality of oligonucleotides are designed such that the difference between the Tm values of the plurality of oligonucleotides for each of the sequences of said regions to be hybridized containing a first genotype of the nucleotide polymorphisms is 0 to 2°C; and
   each of the plurality of oligonucleotides is designed such that the Tm value for the sequence of the region to be hybridized containing the first genotype is different from the Tm value for the sequence of said region to be hybridized containing a second genotype by 3°C or more. The method serves, in particular, to detect whether a sample nucleic acid on which said method is carried out (and which contains the regions containing said plurality of polymorphisms) contains any one or more of said plurality of polymorphisms. In a further embodiment, the polymorphism(s) which is present may be identified.
(2) The method according to (1), wherein the difference between the Tm values of the plurality of oligonucleotides for each of the sequences of said regions to be hybridized containing the first genotype is 0 to 1°C.
(3) A method of detecting nucleotide polymorphisms, comprising, simultaneously or sequentially, performing the nucleotide polymorphism detection method according to (1) or (2) and a nucleotide polymorphism detection method using one or more fluorescent dye detection wavelengths other than and not close to the wavelength to be used in the nucleotide polymorphism detection method according to (1) or (2).
(4) A method of detecting nucleotide polymorphisms, comprising, simultaneously or sequentially, performing the nucleotide polymorphism detection method according to (1) or (2) and a nucleotide polymorphism detection method using two or more fluorescent dye detection wavelengths other than and not close to the wavelength to be used in the nucleotide polymorphism detection method according to (1) or (2).
(5) The method according to any one of (1) to (4), wherein the first genotype-containing sequence is a wild-type sequence and the second genotype-containing sequence is a sequence having a substitution mutation, an insertion mutation, and/or a deletion mutation of one or more nucleotides in the wild-type sequence.
(6) The method according to any one of (1) to (5), wherein the oligonucleotides are nucleotide polymorphism detection probes in which the oligonucleotides emit fluorescence when not hybridized to a target sequence, and fluorescence intensity decreases or increases when the oligonucleotides are hybridized to the target sequence.
(7) The method according to (6), wherein the oligonucleotides are nucleotide polymorphism detection probes in which the fluorescence intensity decreases when the oligonucleotides are hybridized to the target sequence.
(8) A kit for simultaneously detecting in solution a plurality of nucleotide polymorphisms at a single wavelength, the kit comprising nucleotide polymorphism detection probes comprising a plurality of oligonucleotides labeled with a fluorescent dye, each of which hybridizes to a region of a target sequence containing each (i.e. one or at least one) of the plurality of nucleotide polymorphisms, wherein
   the fluorescent dyes labeling the plurality of oligonucleotides are the same or close (similar) to each other in terms of detection wavelength,
   the plurality of oligonucleotides are designed such that the difference between the Tm values of the plurality of oligonucleotides for each of the sequences of said regions to be hybridized containing a first genotype of the nucleotide polymorphisms is 0 to 2°C; and
   each of the plurality of oligonucleotides is designed such that the Tm value for the sequence of the region to be hybridized containing the first genotype is different from the Tm value for the sequence of said region to be hybridized containing a second genotype by 3°C or more.
   The invention also extends to the use of said kit for detecting said plurality of nucleotide polymorphisms at a single wavelength.
(9) A method for predicting medicinal efficacy and/or a physical predisposition in an individual, comprising measuring the presence or absence of a nucleotide polymorphism(s) associated with the medicinal efficacy and/or the physical predisposition, by detecting the plurality of nucleotide polymorphisms by the method according to any one of (1) to (7).
(10) A nucleotide polymorphism detection apparatus comprising:
   a reaction section which contains a plurality of oligonucleotides labeled with a fluorescent dye, each of which hybridizes to a region of a target sequence containing each (i.e. one or at least one) of the plurality of nucleotide polymorphisms;
   a liquid sending section which feeds a sample and/or a reaction solution to the reaction section;
   a light source section which emits light for exciting fluorescence in the reaction section;
   a control section which controls the temperature of the reaction section; and
   a detection section which detects the fluorescence, wherein
   the fluorescent dyes labeling the plurality of oligonucleotides are the same or close to each other in terms of detection wavelength,
   the plurality of oligonucleotides are designed such that the difference between the Tm values of the plurality of oligonucleotides for each of the sequences of said regions to be hybridized containing a first genotype of the nucleotide polymorphisms is 0 to 2°C; and
   each of the plurality of oligonucleotides is designed such that the Tm value for the sequence of the region to be hybridized containing the first genotype is different from the Tm value for the sequence of said region to be hybridized containing a second genotype by 3°C or more.
(11) A nucleotide polymorphism determination system comprising:
   a reaction system which contains a plurality of oligonucleotides labeled with a fluorescent dye, each of which hybridizes to a region of a target sequence containing each (i.e. one or at least one) of the plurality of nucleotide polymorphisms;
   a liquid sending system which feeds a sample and/or a reaction solution to the reaction system;
   a light source system which emits light for exciting fluorescence in the reaction system;
   a control section which controls the temperature of the reaction system;
   a detection system which detects the fluorescence; and
   a determination system which determines the nucleotide polymorphism based on a detection result, wherein
   the fluorescent dyes labeling the plurality of oligonucleotides are the same or close to each other in detection wavelength,
   the plurality of oligonucleotides are designed such that the difference between the Tm values of the plurality of oligonucleotides for each of the sequences of said regions to be hybridized containing a first genotype of the nucleotide polymorphisms is 0 to 2°C; and
   each of the plurality of oligonucleotides is designed such that the Tm value for the sequence of the region to be hybridized containing the first genotype is different from the Tm value for the sequence of said region to be hybridized containing a second genotype by 3°C or more.

The method according to the present invention can detect many nucleotide polymorphisms at one time even when using an apparatus that can detect a limited number of wavelengths and even when there are few kinds of fluorescent molecules that can modify the probes.

### BRIEF DESCRIPTION OF THE DRAWINGS

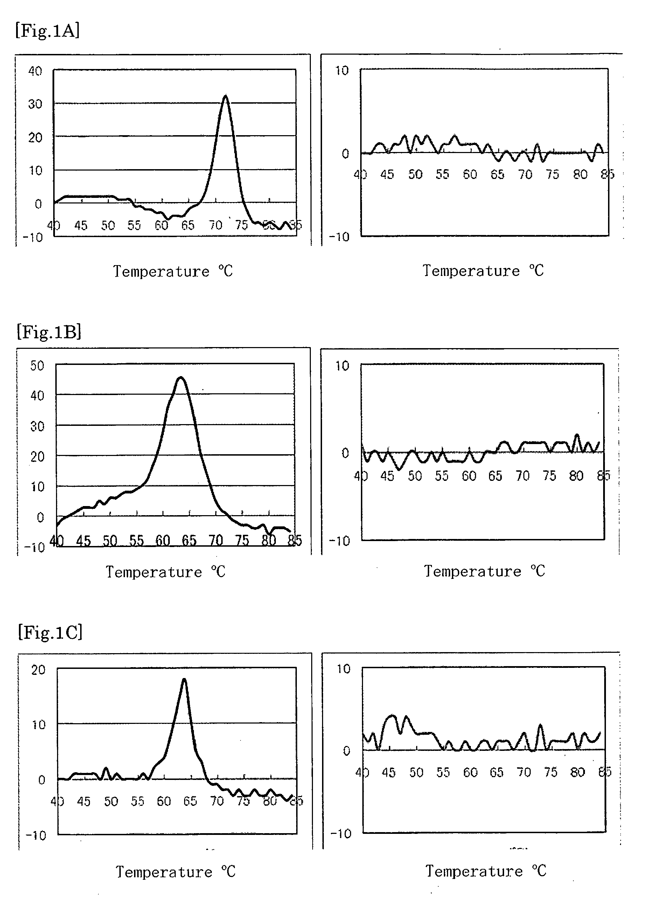

Fig. 1A shows the relationships between variations in the fluorescence intensities of Pacific Blue and BODIPY FL per unit time (d fluorescence intensity increase/t) and temperatures in a Tm analysis as described in Example 1 using probes: 3PB-EGFR-insWT-R5 (SEQ ID NO: 9), 3PB-EGFR-insWT-F4 (SEQ ID NO: 10), and 3PB-EGFR-ins7-F1 (SEQ ID NO: 11), a template: EGFR-e20-ins-WT-F (SEQ ID NO: 1), and fluorescent dyes: Pacific Blue and BODIPY FL. The left-hand side chart shows variations in Pacific Blue and the right-hand side chart shows variations in BODIPY FL. The vertical axes represent the variations in the fluorescence intensities per unit time (d fluorescence intensity increase/t) and the horizontal axes represent temperatures (°C). The drawings below show the results of the same experiments as performed above using different templates.
Fig. 1B shows the results of Example 1 using the template: EGFR-e20-ins9-F (SEQ ID NO: 2).
Fig. 1C shows the results of Example 1 using the template: EGFR-e20-insCAC-R3 (SEQ ID NO: 14).
Fig. 1D shows the results of Example 1 using the template: EGFR-e20-insTGCGTG-F (SEQ ID NO: 4).
Fig. 1E shows the results of Example 1 using the template: EGFR-e20-insAACCCC-R6 (SEQ ID NO: 16).
Fig. 1F shows the results of Example 1 using the template: EGFR-e20-insCCCCAC-R7 (SEQ ID NO: 17).
Fig. 1G shows the results of Example 1 using the template: EGFR-e20-insAACCCCCAC-R8 (SEQ ID NO: 18).
Fig. 1H shows the results of Example 1 using the template: EGFR-e20-insCACGTG-R9 (SEQ ID NO: 19).

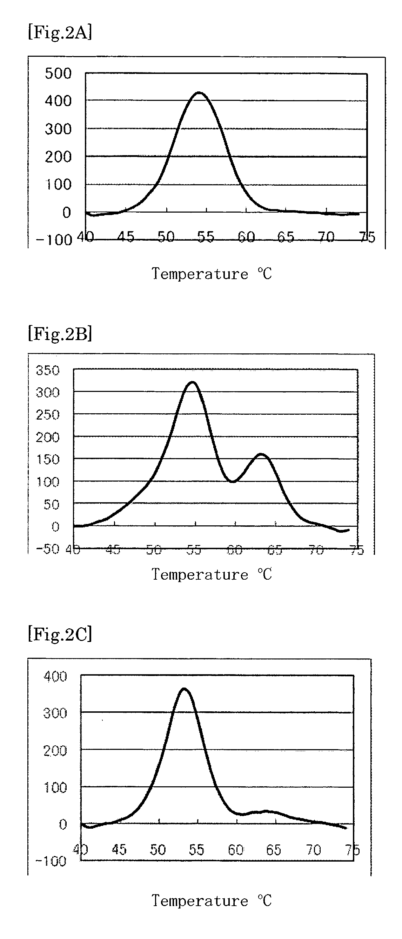

Fig. 2A shows the relationship between variation in the fluorescence intensity of Pacific Blue per unit time (d fluorescence intensity increase/t) and temperature in a Tm analysis as described in Example 2 using probes: 3PB-858-G-PM (SEQ ID NO: 20) and 3PB-3A4-G-PM (SEQ ID NO: 21), a template: 858-mm (SEQ ID NO: 22) 100% and 3A4-mm (SEQ ID NO: 24) 100%, and a fluorescent dye: Pacific Blue. The vertical axis represents the variation in the fluorescence intensity per unit time (d fluorescence intensity increase/t) and the horizontal axis represents temperature (°C). The drawings described below show the results of the same experiments as performed above using different templates.
Fig. 2B shows the results of Example 2 using templates: 858-mm (SEQ ID NO: 22) 50%, 858-PM (SEQ ID NO: 23) 50%, and 3A4-mm (SEQ ID NO: 24) 100%.
Fig. 2C shows the results of Example 2 using templates: 858-mm (SEQ ID NO: 22) 90%, 858-PM (SEQ ID NO: 23) 10%, and 3A4-mm (SEQ ID NO: 24) 100%.
Fig. 2D shows the results of Example 2 using templates: 858-mm (SEQ ID NO: 22) 100%, 3A4-mm (SEQ ID NO: 24) 50%, and 3A4-PM (SEQ ID NO: 25) 50%.
Fig. 2E shows the results of Example 2 using templates: 858-mm (SEQ ID NO: 22) 100%, 3A4-mm (SEQ ID NO: 24) 90%, and 3A4-PM (SEQ ID NO: 25) 10%.

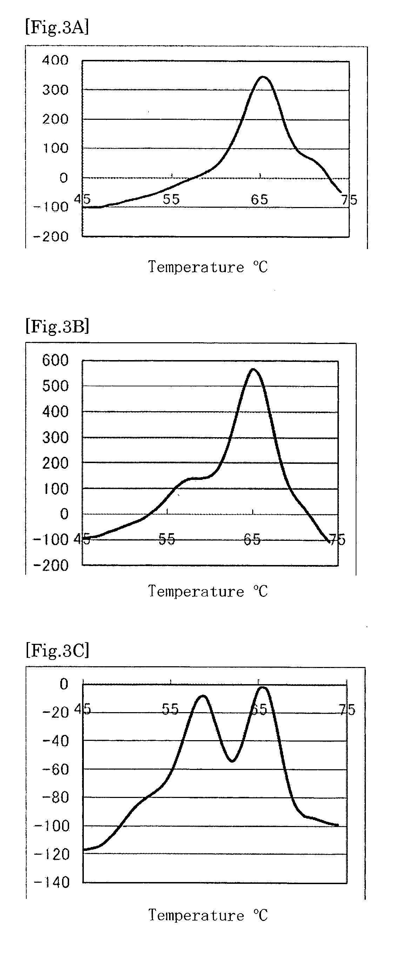

Fig. 3A shows the relationship between variation in the fluorescence intensity of TAMRA per unit time (d fluorescence intensity increase/t) and temperature in a Tm analysis as described in Example 3 using probes: 3T-719-G-PM (SEQ ID NO: 26), 3T-858-G-PM (SEQ ID NO: 27), and 3T-790-T-PM (SEQ ID NO: 28), templates: 719-G (SEQ ID NO: 29) 100%, 858-G (SEQ ID NO: 31) 100%, and 790-T (SEQ ID NO: 33) 100%, and a fluorescent dye: TAMRA. The vertical axis represents the variation in the fluorescence intensity per unit time (d fluorescence intensity increase/t) and the horizontal axis represents temperature (°C). The drawings described below show the results of the same experiments as performed above using different templates.
Fig. 3B shows the results of Example 3 using templates: 719-G (SEQ ID NO: 29) 50%, 719-C (SEQ ID NO: 30) 50%, 858-G (SEQ ID NO: 31) 100%, and 790-T (SEQ ID NO: 33) 100%.
Fig. 3C shows the results of Example 3 using templates: 719-G (SEQ ID NO: 29) 100%, 858-G (SEQ ID NO: 31) 50%, 858-T (SEQ ID NO: 32) 50%, and 790-T (SEQ ID NO: 33) 100%.
Fig. 3D shows the results of Example 3 using templates: 719-G (SEQ ID NO: 29) 100%, 858-G (SEQ ID NO: 31) 100%, 790-T (SEQ ID NO: 33) 50%, and 790-C (SEQ ID NO: 34) 50%.

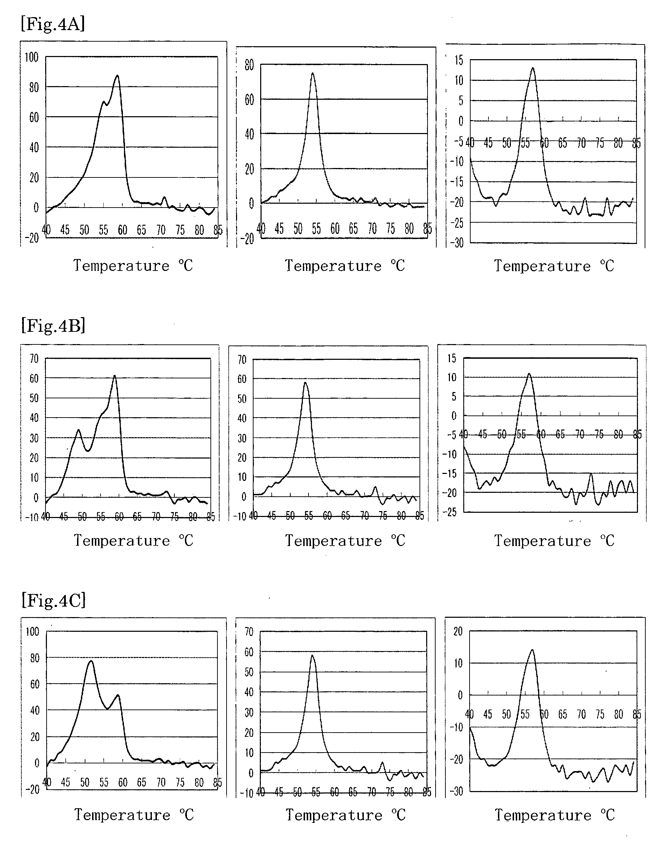

Fig. 4A shows the relationships between variations in the fluorescence intensities of TAMRA, Pacific Blue, and BODIPY FL per unit time (d fluorescence intensity increase/t) and temperatures in a Tm analysis as described in Example 4 using probes: 3T-719-G-PM (SEQ ID NO: 26), 5PB-V12M-A-PM (SEQ ID NO: 35), 3PB-UGT-T-PM (SEQ ID NO: 36), SFL-Q126X-T-PM (SEQ ID NO: 37), and 3FL-NAT-C-PM (SEQ ID NO: 38), templates: V12M-PM (SEQ ID NO: 39) 100%, UGT-PM (SEQ ID NO: 41) 100%, Q126X-PM (SEQ ID NO: 43) 100%, NAT-mm (SEQ ID NO: 46) 100%, and 719-mm (SEQ ID NO: 30) 100%, and fluorescent dyes: TAMRA, Pacific Blue, and BODIPY FL. The left-hand side chart shows variations in Pacific Blue, the central chart shows variations in BODIPY FL, and the right-hand side chart shows variations in TAMRA. The vertical axes represent the variations in the fluorescence intensities per unit time (d fluorescence intensity increase/t) and the horizontal axes represent temperatures (°C). The drawings described below show the results of the same experiments as performed above using different templates.
Fig. 4B shows the results of Example 4 using templates: V12M-PM (SEQ ID NO: 39) 50%, V12M-mm (SEQ ID NO: 40) 50%, UGT-PM (SEQ ID NO: 41) 100%, Q126X-PM (SEQ ID NO: 43) 100%, NAT-mm (SEQ ID NO: 46) 100%, and 719-mm (SEQ ID NO: 30) 100%.
Fig. 4C shows the results of Example 4 using templates: V12M-PM (SEQ ID NO: 39) 100%, UGT-PM (SEQ ID NO: 41) 50%, UGT-mm (SEQ ID NO: 42) 50%, Q126X-PM (SEQ ID NO: 43) 100%, NAT-mm (SEQ ID NO: 46) 100%, and 719-mm (SEQ ID NO: 30) 100%.
Fig. 4D shows the results of Example 4 using templates: V12M-PM (SEQ ID NO: 39) 100%, UGT-PM (SEQ ID NO: 41) 100%, Q126X-PM (SEQ ID NO: 43) 50%, Q126X-mm (SEQ ID NO: 44) 50%, NAT-mm (SEQ ID NO: 46) 100%, and 719-mm (SEQ ID NO: 30) 100%.
Fig. 4E shows the results of Example 4 using templates: V12M-PM (SEQ ID NO: 39) 100%, UGT-PM (SEQ ID NO: 41) 100%, Q126X-PM (SEQ ID NO: 43) 100%, NAT-PM (SEQ ID NO: 45) 50%, NAT-mm (SEQ ID NO: 46) 50%, and 719-mm (SEQ ID NO: 30) 100%.
Fig. 4F shows the results of Example 4 using templates: V12M-PM (SEQ ID NO: 39) 100%, UGT-PM (SEQ ID NO: 41) 100%, Q126X-PM (SEQ ID NO: 43) 50%, Q126X-mm (SEQ ID NO: 44) 50%, NAT-mm (SEQ ID NO: 46) 100%, 719-PM (SEQ ID NO: 29) 50%, and 719-mm (SEQ ID NO: 30) 50%.

### MODES FOR CARRYING OUT THE INVENTION

### <1> Nucleotide Polymorphism Detection Method of the Present Invention

A polymorphism detection method according to the present invention is a method for simultaneously detecting a plurality of nucleotide polymorphisms by melting curve analysis, comprising detecting the plurality of nucleotide polymorphisms at a single wavelength by using a plurality of oligonucleotides each labeled with a fluorescent dye, each of which oligonucleotide hybridizes to a region of a target sequence containing at least one of the plurality of nucleotide polymorphisms, wherein
the detection wavelength of the fluorescent dyes labeling the plurality of oligonucleotides are the same or similar;
the plurality of oligonucleotides are designed such that the difference between the Tm values of the plurality of oligonucleotides for each of the sequences of said regions to be hybridized containing a first genotype of the nucleotide polymorphisms is 0 to 2°C; and
each of the plurality of oligonucleotides is designed such that the Tm value for the sequence of the region to be hybridized containing the first genotype is different from the Tm value for the sequence of said region to be hybridized containing a second genotype by 3°C or more.

Nucleotide polymorphism in the present invention means that the type of nucleotide located at a specific position on a gene exists in multiple forms, and examples of the nucleotide polymorphism include nucleotide substitution, insertion and deletion of one or more nucleotides.

The term "a plurality of nucleotide polymorphisms" in the present invention includes both cases of detecting a plurality of nucleotide polymorphisms in a single gene and detecting a single nucleotide polymorphism in each of a plurality of genes.

The method according to the present invention uses a plurality of oligonucleotides labeled with a fluorescent dye that is the same or close to each other in terms of detection wavelength, each of which hybridizes to a nucleotide polymorphism-containing region.

An exemplary case of using two kinds of oligonucleotides will be now described. Oligonucleotide 1 is hybridized to a first polymorphic region and oligonucleotide 2 is hybridized to a second polymorphic region. A fluorescent dye labeling oligonucleotide 1 and a fluorescent dye labeling oligonucleotide 2 are the same or close to each other in detection wavelength.

As used herein, the terms "close" or "similar" refer to a wavelength range subjected to interference that affects measurement results upon detection of a fluorescence value. Such a close wavelength range varies depending on the combination of fluorescent dyes, and the difference between detection wavelengths (maximum wavelengths) is, for example, within (i.e. less than) 200 nm, within 100 nm, within 50 nm, or within 30 nm. Examples of the combination of fluorescent dyes mutually close in detection wavelength as above include BODIPY FL and FAM, BODIPY FL and TRITC, Pacific Blue and Alexa Fluor 405, TAMRA and Alexa Fluor 670, TAMRA and Alexa Fluor 700, and TAMRA and Texas Red. That is, in the method according to the present invention, even when the same fluorescent dye is not necessarily used, labeling each of the oligonucleotides with a fluorescent dye close to each other in detection wavelength allows for the simultaneous detection of a plurality of nucleotide polymorphisms at a single wavelength.

The single wavelength in the present invention means not only the same wavelength but also the close or similar wavelength range as mentioned above.

In the case of having the same detection wavelength this usually means that a plurality of oligonucleotides is labeled with the same fluorescent dye.

In the method according to the present invention, as long as the detection of a plurality of nucleotide polymorphisms is performed at a single wavelength, the detection of nucleotide polymorphisms (those different from the plurality of nucleotide polymorphisms) using one or two or more kinds of probes labeled with a fluorescent dye having one or two or more other detection wavelengths (other than and not close to the "single wavelength" to be used for detecting the "plurality of the nucleotide polymorphisms") may be simultaneously or sequentially performed. That is, as long as a plurality of nucleotide polymorphisms is detected at a single wavelength, the detection of other nucleotide polymorphisms at one or two or more other wavelengths may be simultaneously or sequentially performed.

In the method according to the present invention, the Tm values of the plurality of oligonucleotides for each of the sequences of the regions to be hybridized containing a first genotype are concentrated in a close range. As used herein, regarding the close temperature range, the difference between the respective Tm values for the first genotype-containing sequences of the plurality of oligonucleotides is 0 to 2°C, or for example 0 to 1°C.

Furthermore, each of the plurality of oligonucleotides is designed such that the Tm value for the sequence of the region to be hybridized containing the first genotype is different from the Tm value for the sequence of said region to be hybridized containing a second genotype by 3°C or more, 5°C or more, or 10°C or more, for example.

Now, a description will be given of an exemplary case of using two oligonucleotides: oligonucleotide 1 hybridized to a first polymorphic region and oligonucleotide 2 hybridized to a second polymorphic region (alternatively, three or more kinds of oligonucleotides may be used).

The temperature difference between the Tm value of oligonucleotide 1 for a first genotype (such as a wild-type) of the first polymorphic region and the Tm value of oligonucleotide 2 for the first genotype (such as a wild-type) of the second polymorphic region is 0 to 2°C, or for example 0 to 1°C.

Then, the temperature difference between the Tm value of oligonucleotide 1 for the first genotype (such as a wild-type) of the first polymorphic region and the Tm value of oligonucleotide 1 for the second genotype (such as a mutant type) of the first polymorphic region is 3°C or more, 5°C or more, or 10°C or more, for example.

Furthermore, the temperature difference between the Tm value of oligonucleotide 2 for the first genotype (such as a wild-type) of the second polymorphic region and the Tm value of oligonucleotide 2 for a second genotype (such as a mutant type) of the second polymorphic region is 3°C or more, 5°C or more, or 10°C or more, for example.

The temperature difference between the Tm value of oligonucleotide 1 for the second genotype (such as a mutant type) of the first polymorphic region and the Tm value of oligonucleotide 2 for the second genotype (such as a mutant type) of the second polymorphic region may be 0°C, but is advantageously 1°C or more so as to be able to distinguish both mutations, for example.

When the first genotype-containing sequence is a wild-type sequence and the second genotype-containing sequence is a mutant-type sequence having one or more nucleotide substitution mutations, insertion mutations and/or deletion mutations in a wild-type sequence, e.g. the corresponding wild-type sequence, by using a setting such that peaks of the wild-type sequence for the plurality of the oligonucleotides are concentrated in a predetermined temperature range, mutant-type sequences can be detected without detection of the peaks of the wild-type sequence.

That is, since Tm values for the wild-type are encompassed in the predetermined temperature range, the presence or absence of a mutant type can be detected by detecting a peak shifted from the Tm value of the wild-type, which corresponds to the mutant type.

In other words, by concentrating in advance the peaks (namely, Tm values) obtained in melting curve analysis regarding the first genotype-containing sequence in a predetermined temperature range, a mutation can be detected by measuring a peak of the second genotype-containing sequence (preferably, as described above, a peak shifted by 3°C or more from the peak of the first genotype-containing region), without measurement of any peak in the melting curve analysis regarding the first genotype-containing sequence.

For example, it is desirable that, as the oligonucleotides, a sequence is selected which is completely (i.e. 100%) matching (i.e. complementary) with the first genotype (wild-type)-containing sequence but including a mismatch with respect to the second genotype (mutant type)-containing sequence, and as described above, the oligonucleotides are designed such that the Tm value for the first genotype-containing sequence is higher than the Tm value for the second genotype-containing sequence by 3°C or more, 5°C or more, or 10°C or more, for example.

Alternatively, probe sequences may be selected that include a mismatch with respect to the first genotype (wild-type)-containing sequence but completely (i.e. 100%) match (i.e. are complementary) with the second genotype (mutant type)-containing sequence and may be designed such that the Tm value for the first genotype-containing sequence is lower than the Tm value for the second genotype-containing sequence by 3°C or more, preferably 5°C or more, and more preferably 10°C or more.

The first genotype and the second genotype may both be mutants. When the first genotype-containing sequence and the second genotype-containing sequence are both mutant sequences, detection can also determine whether the second genotype-containing sequence includes more mutations than the first genotype-containing sequence.

As the oligonucleotides to be hybridized to the nucleotide polymorphism-containing regions and used in the method according to the present invention, for example, a quenching probe with fluorescent dye-labeled end(s) can be used, as described in JP2001-86300A and JP2002-119291A. In such an oligonucleotide probe, when hybridized to the target sequence, for example, fluorescence of the fluorescent dye decreases or increases compared to when the probe is not hybridized to a target sequence. The oligonucleotide probe in the present invention is, for example, a quenching probe in which fluorescence of the fluorescent dye is emitted when the probe is not hybridized, whereas the fluorescence thereof is quenched when it is hybridized.

The length of oligonucleotide probes to be used in the present invention is not specifically limited, but the probes are, for example, 40 nucleotides or less in length to obtain the difference of Tm values between the first genotype-containing sequence and the second genotype-containing sequence, and may be for example, 11 to 40 nucleotides in length, 12 to 30 nucleotides in length, 15 to 30 nucleotides in length, or 18 to 30 nucleotides in length.

Examples of nucleotide sequences of the probes for detecting nucleotide polymorphisms to be used in the present invention include probes shown in SEQ ID NOs: 9 to 11, SEQ ID NOs: 20 to 21, SEQ ID NOs: 26 to 28, and SEQ ID NOs: 35 to 38.

As the fluorescent dyes to be used, those described in JP2001-286300A, JP2002-119291A, and the like, may be used. Specific examples of the fluorescent dyes include Pacific Blue, FAM, TAMRA, and BODIPY FL. The method for binding a fluorescent dye to the oligonucleotides can be performed according to a usual method, such as any of methods described in JP2001-286300A and JP2002-119291A. A "usual" method as used herein refers to any method known in the art and of which the skilled person is familiar.

The method according to the present invention is a method using the above-described oligonucleotide probes, and analyzes nucleotide polymorphisms by melting curve analysis.

The method according to the present invention can be performed according to a usual melting curve analysis method (Tm analysis), except that a plurality of oligonucleotides labeled with a fluorescent dye each of which is the same or close to each other in detection wavelength is used; the plurality of oligonucleotides are designed such that the difference between the Tm values for the sequence containing a first genotype of the plurality of nucleotide polymorphisms is 0 to 2°C; and each of the plurality of oligonucleotides is designed such that the Tm value for the first genotype-containing sequence is different from the Tm value for the second genotype-containing sequence by 3°C or more. Melting curve analysis can be performed, for example, according to the methods described in JP2001-286300A and JP2002-119291A.

In melting curve analysis, when a mutant peak is detected, the presence of a mutation can be concluded.

In addition, the method according to the present invention may include amplifying a nucleic acid before the detection of nucleotide polymorphisms or simultaneously with the detection of nucleotide polymorphisms.

The nucleic acid amplification method is, for example, a method using polymerase, and examples of the method include PCR, ICAN, and LAMP. When amplification is performed using polymerase, for example, amplification is performed in the presence of the probes according to the present invention. According to the probe to be used, it is easy for those skilled in the art to adjust reaction conditions for amplification and the like. Thereby, it is only necessary to analyze the Tm values of probes after amplification of a nucleic acid, so that purification or the like of an amplification product after completion of the reaction is unnecessary. Therefore, there is no worry about contamination due to the amplification product. Additionally, since the detection of nucleotide polymorphisms can be performed by the same apparatus as that necessary for amplification, transfer of the container is not needed, so that automation of the process can be easily achieved.

In the present invention, DNA present in a sample may be single-stranded DNA or double-stranded DNA. When the DNA is double-stranded DNA, for example, the method may include a step of separating the double-stranded DNA in the sample by heating before the hybridization step. By separating the double-stranded DNA into single-stranded DNA, hybridization to the detection probes can be performed in the next hybridization step.

The nucleic acid used as a template for nucleic acid amplification is not specifically limited as long as it includes nucleic acid, and for example, may be one derived or derivable from arbitrary biological sources such as blood, an oral mucosa suspension, a somatic cell such as nail or hair, a germ cell, milk, ascitic fluid, paraffin embedded tissue, gastric juice, gastric lavage fluid, peritoneal fluid, amniotic fluid, and cell culture. The nucleic acid as the template can be used directly as it is obtained from any of the sources or after performing pretreatment to alter the properties of the sample, e.g. amplification of the nucleic acid.

Tm analysis can be performed according to a usual method, except for measuring fluorescence of the fluorescent dye(s) of the probes in the present invention. The measurement of fluorescence can be performed by measuring light having a light emission wavelength using excitation light with a wavelength according to the fluorescent dye(s). The rate of temperature rise in Tm analysis is usually 0.1 to 1°C/second. The composition of the reaction solution used to perform Tm analysis is not specifically limited as long as it allows for the hybridization of a probe to a nucleic acid having a sequence complementary to a nucleotide sequence of the probe, but usually, the concentration of monovalent cation in the solution is 1.5 to 5 mM and the pH is 7 to 9. Since the reaction solution for an amplification method using DNA polymerase, such as PCR, meets those conditions, the post-amplification reaction solution can be used as it is in Tm analysis.

Detection of nucleotide polymorphisms based on the results of Tm analysis can be performed according to a usual method. Detection in the present invention includes detection of the presence or absence of mutation and the amount of mutation.

The nucleotide polymorphism detection method according to the present invention can detect nucleotide polymorphism(s) and can determine the presence or absence of nucleotide polymorphism(s) associated with a medicinal effect and/or physical predisposition to predict the medicinal effect on an individual and/or the physical predisposition of the individual. For example, in the case of an EGFR exon 20 insertion polymorphism, the efficacy of gefitinib as an anticancer drug can be determined. The EGFR exon 20 insertion polymorphism may be detected in a nucleic acid sample, e.g. from a biological source as described above, taken from an individual, i.e. subject, e.g. a human.

### <2> Kit of the Present Invention

A kit according to the present invention is a kit used for the nucleotide polymorphism detection method according to the invention.

Specifically, the kit of the present invention is a kit for simultaneously detecting in solution a plurality of nucleotide polymorphisms at a single wavelength, comprising nucleotide polymorphism detection probes consisting of a plurality of oligonucleotides each labeled with a fluorescent dye each of which is the same or close to each other in terms of detection wavelength. The kit is characterized in that the plurality of oligonucleotides are designed such that the difference between the Tm values of the plurality of oligonucleotides for each of the sequences of said regions to be hybridized containing a first genotype of the nucleotide polymorphisms is 0 to 2°C; and each of the plurality of oligonucleotides is designed such that the Tm value for the sequence of the region to be hybridized containing the first genotype is different from the Tm value for the sequence of said region to be hybridized containing a second genotype by 3°C or more.

The detection kit according to the present invention may further include, in addition to the above probes, primers for amplification using reagents required to perform nucleic acid amplification, particularly DNA polymerase.

In the detection kit according to the present invention, the probes, the primers, and other reagents may be separately contained or parts thereof may be combined in a mixture.

### <3> Apparatus of the Present Invention

An apparatus according to the present invention is an apparatus used for the nucleotide polymorphism detection method, and specifically, includes a reaction section that contains a plurality of oligonucleotides which hybridize to a nucleotide polymorphism-containing region in a target sequence, a liquid sending section that feeds a sample and/or reaction solution to the reaction section, a light source section that emits light for exciting fluorescence to the reaction section, a control section that controls the temperature of the reaction section, and a detection section that detects the fluorescence. The apparatus is characterized in that, as described in <1> the nucleotide polymorphisms detection method according to the present invention, the plurality of oligonucleotides are each labeled with a fluorescent dye each of which is the same or close to each other in terms of detection wavelength; the plurality of oligonucleotides are designed such that the difference between the Tm values of the plurality of oligonucleotides for each of the sequences of said regions to be hybridized containing a first genotype of the nucleotide polymorphisms is 0 to 2°C; and each of the plurality of oligonucleotides is designed such that the Tm value for the sequence of the region to be hybridized containing the first genotype is different from the Tm value for the sequence of said region to be hybridized containing a second genotype by 3°C or more.

In the reaction section, the plurality of oligonucleotides may be fixed or a substrate with the plurality of oligonucleotides fixed thereon may be formed immediately before its use.

The liquid sending section may simultaneously or separately send a gene-containing sample and a reaction solution. In addition, the liquid sending section may be adapted such that cleansing liquid can be sent.

The light source section can be of any form as long as it is a light source capable of emitting light having an excitation wavelength corresponding to the oligonucleotide-labeling fluorescent dye. When simultaneously or sequentially performing the detection of nucleotide polymorphisms (those different from the plurality of nucleotide polymorphisms) using one or two or more kinds of probes labeled with a fluorescent dye having one or two or more other detection wavelengths (other than and not close to the "single wavelength" to be used for detecting the "plurality of the nucleotide polymorphisms"), one or two or more kinds of light sources capable of emitting light having other excitation wavelength(s) may be combined.

For example, the control section can increase or decrease the temperature of the reaction section at a predetermined rate and also can maintain it at a predetermined temperature.

The detection section can be of any form as long as it can detect at the same or a close detection wavelength corresponding to the fluorescent dye labeling the oligonucleotides. When simultaneously or sequentially performing the detection of nucleotide polymorphisms (those different from the plurality of nucleotide polymorphisms) using one or two or more kinds of probes labeled with a fluorescent dye having one or two or more other detection wavelengths (other than and not close to the "single wavelength" to be used for detecting the "plurality of the nucleotide polymorphisms"), the other detection wavelengths may be detected. In addition, only the second genotype may be assigned as a detection target and only the peak of a second genotype-containing sequence (for example, a peak shifted from the peak of a first genotype-containing sequence by 3°C or more) may be detected.

### <4> System of the Present Invention

A system according to the present invention is a system used for a nucleotide polymorphism detection method, and specifically includes a reaction system that contains a plurality of oligonucleotides which hybridize to a nucleotide polymorphism-containing region in a target sequence, a liquid sending system that feeds a reagent and/or reaction solution to the reaction system, a light source section that emits light for exciting fluorescence in the reaction system, a control section that controls temperature of the reaction system, a detection section that detects fluorescence, and a determination system that determines a nucleotide polymorphism based on a detection result. The system of the present invention is characterized in that, as described in <1> the nucleotide polymorphism detection method according to the present invention, the plurality of oligonucleotides are each labeled with a fluorescent dye each of which is the same or close to each other in detection wavelength; the plurality of oligonucleotides are designed such that the difference between the Tm values of the plurality of oligonucleotides for each of the sequences of said regions to be hybridized containing a first genotype of the nucleotide polymorphisms is 0 to 2°C; and each of the plurality of oligonucleotides is designed such that the Tm value for the sequence of the region to be hybridized containing the first genotype is different from the Tm value for the sequence of said region to be hybridized containing a second genotype by 3°C or more.

For the reaction system, the liquid sending system, the light source system, the control system, and the detection system, the apparatus according to the present invention can be used. In addition, the determination system may be like a computer connected to the apparatus according to the present invention to output the determination of the type of nucleotide polymorphism, the presence or absence of mutation, and the like from input detection results.

### EXAMPLES

Hereinafter, the present invention will be described in detail with reference to Examples, but the invention is not limited to the following Examples.

### Example 1 (Detection of template oligonucleotides with EGFR exon 20 insertion by three kinds of probes at two wavelengths)

Regarding EGFR exon 20 insertion, besides a wild-type (hereinafter referred to as WT), the following seven insertion mutations are known. Since the efficacy rate of gefitinib on patients with EGFR exon 20 insertion mutations is said to be 0%, it is necessary to detect an EGFR exon 20 insertion mutant type (hereinafter referred to as mt).

The sequence shown in SEQ ID NO: 1 represents nucleotide numbers 2530 - 2579 in NM_005228.3 GENE ID: 1956.

**[Table 1]**

| | Name | Sequence (5'→ 3') | SEQ ID NO. |
|---|---|---|---|
| WT | EGFR-e20-ins-WT-F | Gaagcctacgtgatggccagcgtggacaacccccacgtgtgccgcctgct | SEQ ID NO: 1 |
| mt1 | EGFR-e20-ins9-F | gaagcctacgtgatggccagcgtgGCCAGCGTGgacaacccccacgtgtgccgcctgct | SEQ ID NO: 2 |
| mt2 | EGFR-e20-insCAC-F | gaagcctacgtgatggccagcgtggacaacccccacCACgtgtgccgcctgct | SEQ ID NO: 3 |
| mt3 | EGFR-e20-insTGCGTG-F | gaagcctacgtgatggccagcgtgTGCGTGgacaacccccacgtgtgccgcctgct | SEQ ID NO: 4 |
| mt4 | EGFR-e20-insAACCCC-F | gaagcctacgtgatggccagcgtggacaaccccAACCCCcacgtgtgccgcctgct | SEQ ID NO: 5 |
| mt5 | EGFR-e20-insCCCCAC-F | gaagcctacgtgatggccagcgtggacaacccccacCCCCACgtgtgccgcctgct | SEQ ID NO: 6 |
| mt6 | EGFR-e20-insAACCCCCAC-F | gaagcctacgtgatggccagcgtggacaacccccacAACCCCCACgtgtgccgcctgct | SEQ ID NO: 7 |
| mt7 | EGFR-e20-insCACGTG-F | gaagcctacgtgatggccagcgtggacaacccccacgtgCACGTGtgccgcctgct | SEQ ID NO: 8 |

| | | | |
|---|---|---|---|
| * Nucleotides indicated by capital letters represent mutation sites. | | | |

To cover all mt types using a method described in JP2002-355084A, it is necessary to use long probes of approximately 40 bp in length, but, in this manner, a Tm value difference may not be obtained and thus mutation detection may be prevented. Accordingly, some probes perfectly matching with a WT and having the same Tm value as that of the WT were prepared, and fluorophores of the probes were made so as to have the same wavelength.

Based on a nucleotide sequence (SEQ ID NO: 1 (wild-type)) containing an EGFR exon 20 insertion polymorphism site, probes having C at their 3' end ((wild-type: SEQ ID NOs: 9 and 10) and (mutant type: SEQ ID NO: 11)) were designed, as shown in Table 2 below. In Table 2, the position of each probe is represented by nucleotides numbered relative to the nucleotide sequence shown in SEQ ID NO: 1 in respect of SEQ ID NOs: 9 and 10, and in SEQ ID NO: 7 in respect of SEQ ID NO: 11. Labeling with Pacific Blue or BODIPY FL was performed according to a usual method.

Additionally, Table 2 shows the sequences of a template oligonucleotide (a wild-type antisense (SEQ ID NO: 12)) and mutant type antisense oligonucleotides (SEQ ID NOs: 13 to 19) used as detection targets. The sequences shown in SEQ ID NOs: 12 to 19, respectively, are the complete complementary strands of SEQ ID NOs: 1 to 8, respectively.

The probe of SEQ ID NO: 9 can detect mt1 and mt3 of Table 1; the probe of SEQ ID NO: 10 can detect mt2, mt4, mt5, and mt7 of Table 1; and the probe of SEQ ID NO: 11 can detect mt6 of Table 1.

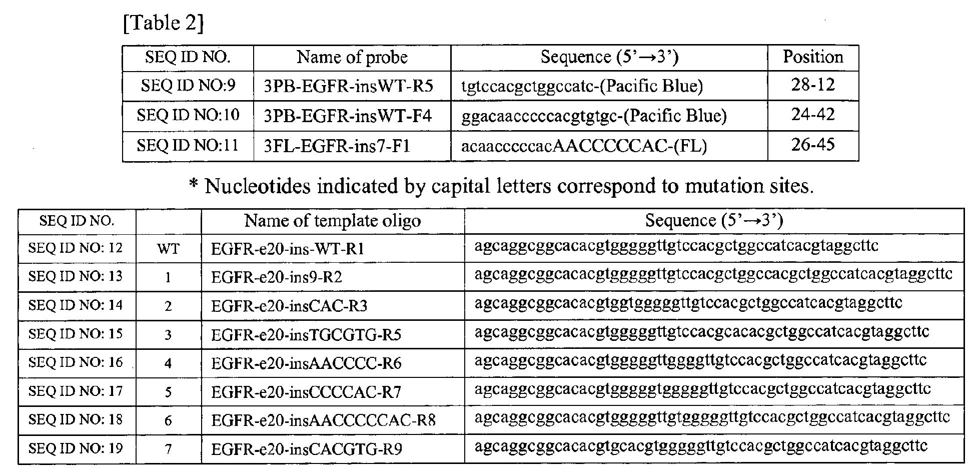

Tm analysis was performed using a fully automatic SNPs analyzer (product name: i-densy IS-5310 manufactured by ARKRAY, Inc.). Also in the following Tm analysis, the same analyzer was used unless specifically stated otherwise. The composition of the probe solution is as shown below. Samples used were template oligonucleotides as shown below. Conditions for the Tm analysis were set to: 95°C, 1 sec. → 40°C, 60 sec. → (40°C → 85°C, 1°C/3 sec.).

Excitation wavelengths and detection wavelengths, respectively, in the Tm analysis were set to 365 to 415 nm and 445 to 480 nm (Pacific Blue (PB)), respectively, or 420 to 485 nm and 520 to 555 nm (BODIPY FL (FL)), respectively.

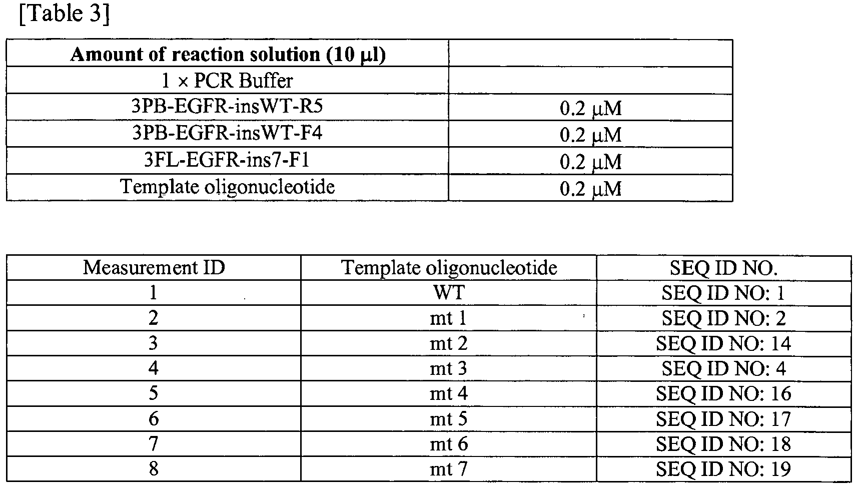

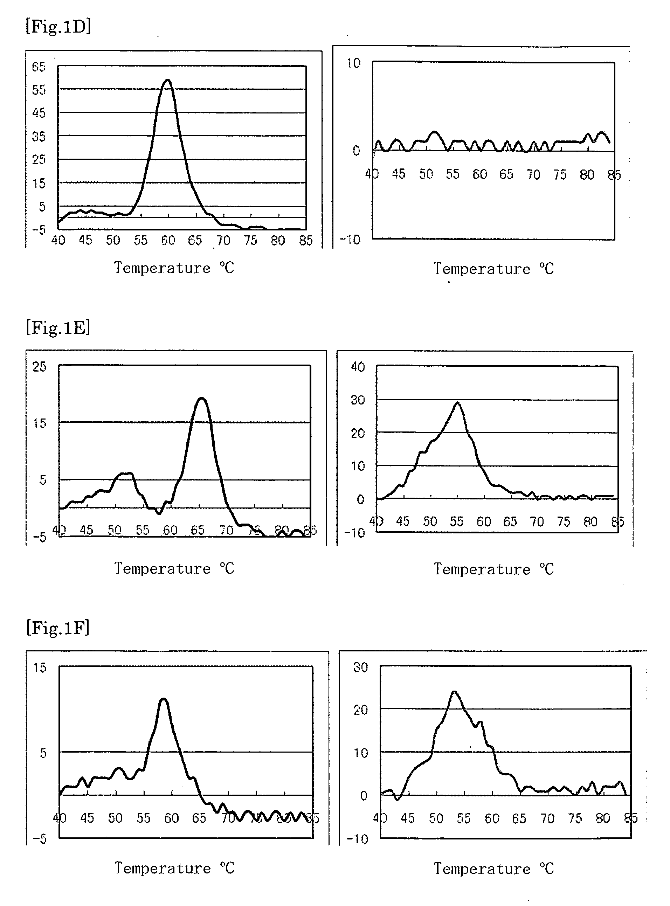

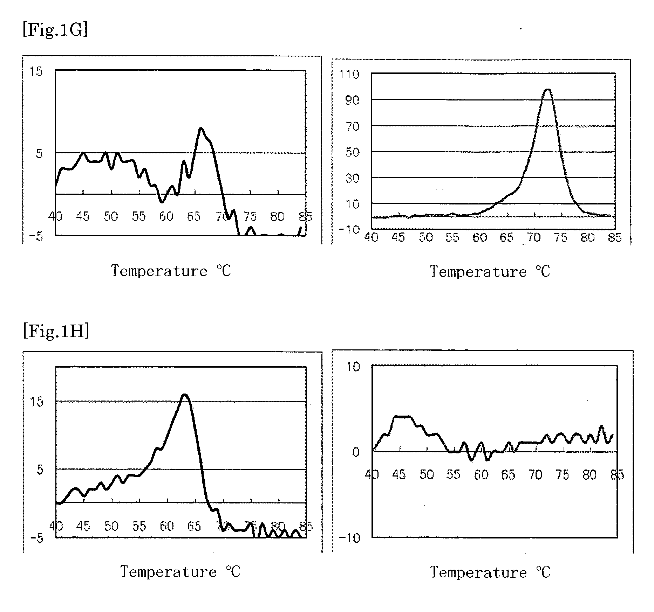

In the results of the Tm analysis using the probes of Table 2 and the template oligonucleotides of Table 3, clear mutant peaks of Pacific Blue were observed in WT, mt1, mt2, mt3, mt4, mt5, and mt7 (left-hand side charts of Figs. 1A, 1B, 1C, 1D, 1E, 1F, and 1H), whereas no mutant peak of Pacific Blue was observed in mt6 (left-hand side chart of Fig. 1 G). In addition, mt6 exhibited mutant peaks of BODIPY FL (right-hand side chart of Fig. 1G), whereas WT, mt 1, mt 2, mt 3, mt 4, mt 5, and mt 7 exhibited no clear mutant peak of BODIPY FL (right-hand side charts of Figs. 1A, 1B, 1C, 1D, 1E, 1F, and 1H).

In other words, for all of mt1 to mt7, peaks different from the wild-type peak (72°C) were able to be detected, and thus the detection of mutations of mt1 to mt7 is possible.

Table 4 below shows the results.

**[Table 4]**

| Measurement ID | | PB probe (Tm) | FL probe (Tm) |
|---|---|---|---|
| 1 | WT | 72 | |
| 2 | mt1 | 64 | |
| 3 | mt2 | 64 | |
| 4 | mt3 | 60 | |
| 5 | mt4 | 66 | |
| 6 | mt5 | 59 | |
| 7 | mt6 | | 73 |
| 8 | mt7 | 63 | |

From the above, using two kinds of probes: 3PB-EGFR-insWT-R5 and 3PB-EGFR-insWT-F4, the plurality of mutations were able to be detected at a single wavelength: 445 to 480 nm. Then, by combining with 3FL-EGFR-ins7-F1, all EGFR exon 20 insertion mutations were able to be detected.

### Example 2 (Detection of template oligonucleotides of two different kinds of genes using two kinds of probes at a single wavelength)

As shown in Table 5, a probe (corresponding to 3PB-858-G-PM (SEQ ID NO: 20) was designed having a sequence complementary to the nucleotide sequence of SEQ ID NO: 23 and having C at its end. In Table 5, the position of the probe is represented by nucleotides numbered relative to the nucleotide sequence shown in SEQ ID NO: 23. A perfect matching type, which will be hereinafter referred to as PM type (SEQ ID NO: 23), as a template oligonucleotide used as a detection target, and a mismatching type, which will be hereinafter referred to as mm type (SEQ ID NO: 22), as a non-detection target, were also designed.

In addition, as shown in Table 5, a probe (corresponding to 3PB-3A4-G-PM (SEQ ID NO: 21) was designed having a sequence complementary to the nucleotide sequence of SEQ ID NO: 25 and having C at its end. In Table 5, the position of the probe is represented by nucleotides numbered relative to the nucleotide sequence shown in SEQ ID NO: 25. A PM type (SEQ ID NO: 25) as a template oligonucleotide used as a detection target and an mm type (SEQ ID NO: 24) as a non-detection target were also designed.

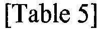

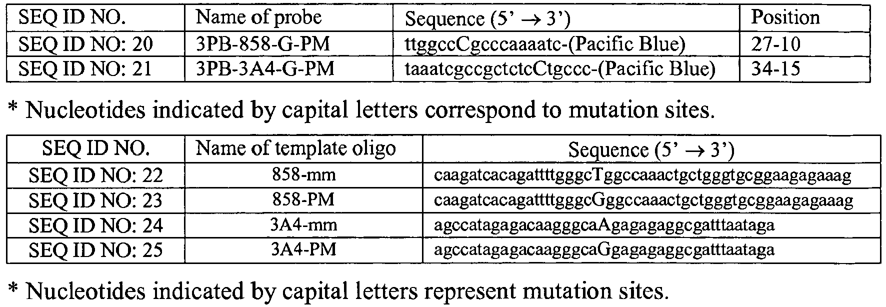

The composition of the PCR reaction solution is as shown below. The samples used were the combinations of template oligonucleotides as below. Conditions for the Tm analysis were set to: 95°C, 1 sec. → 40°C, 60 sec. → (40°C→ 75°C, 30°C/1 sec.).

Excitation wavelengths and detection wavelengths, respectively, in the Tm analysis were set to 365 to 415 nm and 445 to 480 nm (Pacific Blue), respectively.

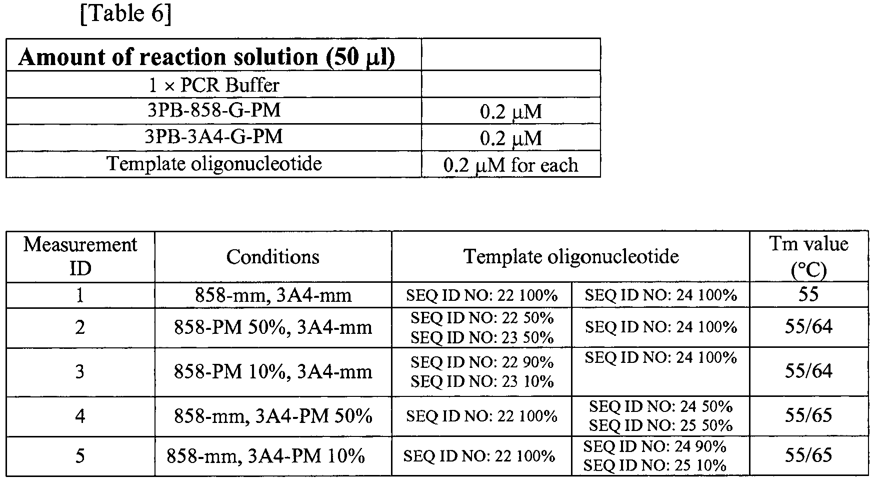

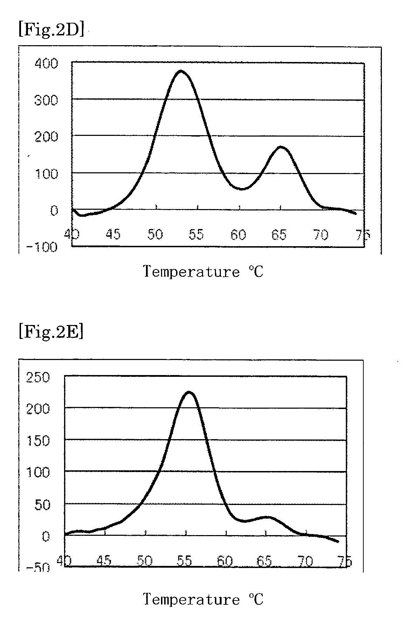

In the results of the Tm analysis using the probes of Table 5, regardless of including 50% of the nucleotide sequences (SEQ ID NOs: 23 and 25) as the detection targets (Figs. 2B and 2D) and 10% thereof (Figs. 2C and 2E), clear PM-type peaks, in addition to mm-type peaks, were observed.

In other words, the peaks different from the mm-type peaks (55°C) were able to be detected, and thus detection of the PM types as the detection targets at a sensitivity of 10% is possible.

### Example 3 (Detection of template oligonucleotides of three different kinds of genes using three kinds of probes at a single wavelength)

As shown in Table 7, probes (corresponding to 3T-719-G-PM, 3T-858-G-PM, and 3T-790-T-PM (SEQ ID NOs: 26 to 28)) were designed having complementary sequences to three nucleotide sequences (SEQ ID NOs: 29, 31, and 33) and having C at the 3' ends thereof. In Table 7, the positions of the probes are each represented by nucleotides numbered relative to the nucleotide sequence shown in each of SEQ ID NOs: 29, 31, and 33. In addition, mm types (SEQ ID NOs: 30, 32, and 34) as template oligonucleotides used as detection targets and PM types (SEQ ID NOs: 29, 31, and 33) as non-detection targets, were designed.

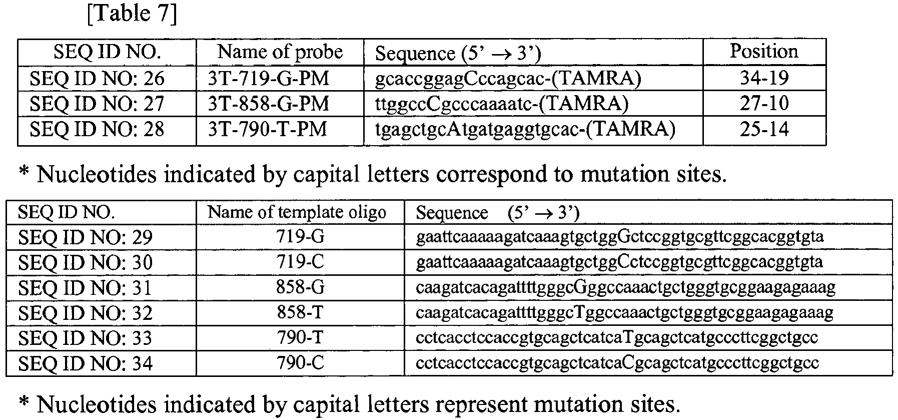

The composition of the PCR reaction solution is as shown below. The samples used were combinations of template oligonucleotides as below. Conditions for the Tm analysis were set to: 95°C, 1 sec. → 40°C, 60 sec. → (40°C →75°C, 30°C/1 sec.).

Excitation wavelengths and detection wavelengths, respectively, in the Tm analysis were set to 520 to 555 nm and 585 to 700 nm (TAMRA (T)), respectively.

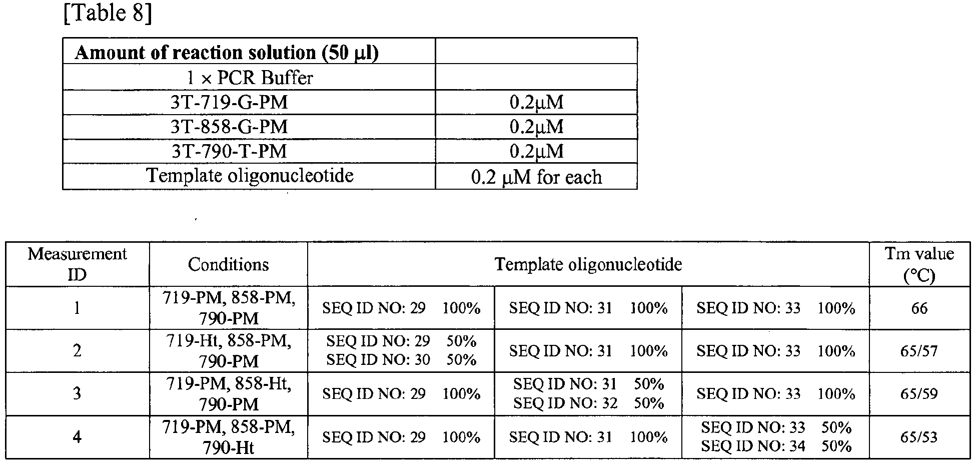

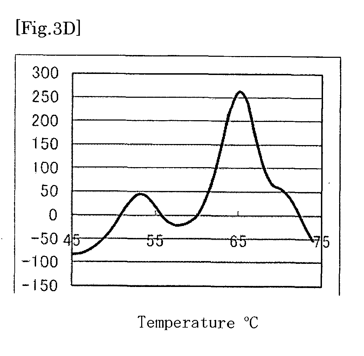

In the results of the Tm analysis using the probes of Table 7, in the case of including 50% of nucleotide sequences (SEQ ID NOs: 30, 32, and 34) as the detection targets (Figs. 3B, 3C, and 3D), clear mm-type peaks in addition to PM-type peaks were observed.

In other words, the peaks different from the wild-type peak (65 to 66°C) were able to be detected, and thus detection of the three different kinds of polymorphisms is possible.

### Example 4 (Detection of template oligonucleotides of five different types of genes using five kinds of probes at three wavelengths)

As shown in Table 9, probes (corresponding to 3T-719-G-PM, 5PB-V12MA-PM, 3PB-UGT-T-PM, 5FL-Q126X-T-PM, and 3FL-NAT-C-PM (SEQ ID NOs: 26, and 35 to 38)) were designed having sequences complementary to five nucleotide sequences (SEQ ID NOs: 29, 39, 41, 43, and 45) and having C at their end. In Table 9, the positions of the probes are each represented by nucleotides numbered relative to the nucleotide sequences shown in SEQ ID NOs: 29, 39, 41, 43, and 45. PM types (SEQ ID NOs: 39,41, and 43) and mm types (SEQ ID NOs: 30 and 46) as template oligonucleotides used as detection targets, and mm types (SEQ ID NOs: 40, 42, and 44) and PM types (SEQ ID NOs: 29 and 45) as non-detection targets, were also designed.

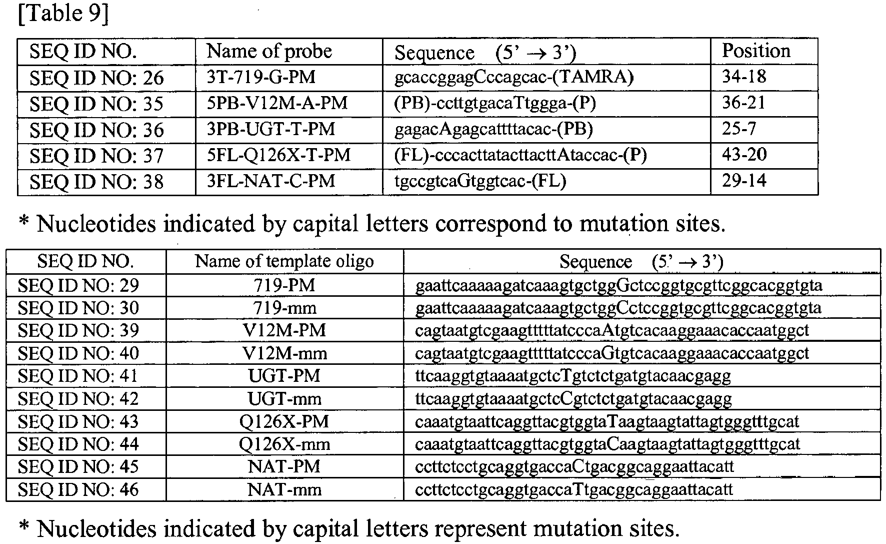

The composition of the PCR reaction solution is as shown below. The samples used were combinations of template oligonucleotides as shown below. Conditions for the Tm analysis were set to: 95°C, 1 sec. → 40°C, 60 sec. → (40°C→ 85°C, 30°C/1 sec.).

Excitation wavelengths and detection wavelengths, respectively, in the Tm analysis were set to 365 to 415 nm and 445 to 480 nm (Pacific Blue), respectively, 420 to 485 nm and 520 to 555 nm (BODIPY FL), respectively, or 520 to 555 nm and 585 to 700 nm (TAMRA), respectively.

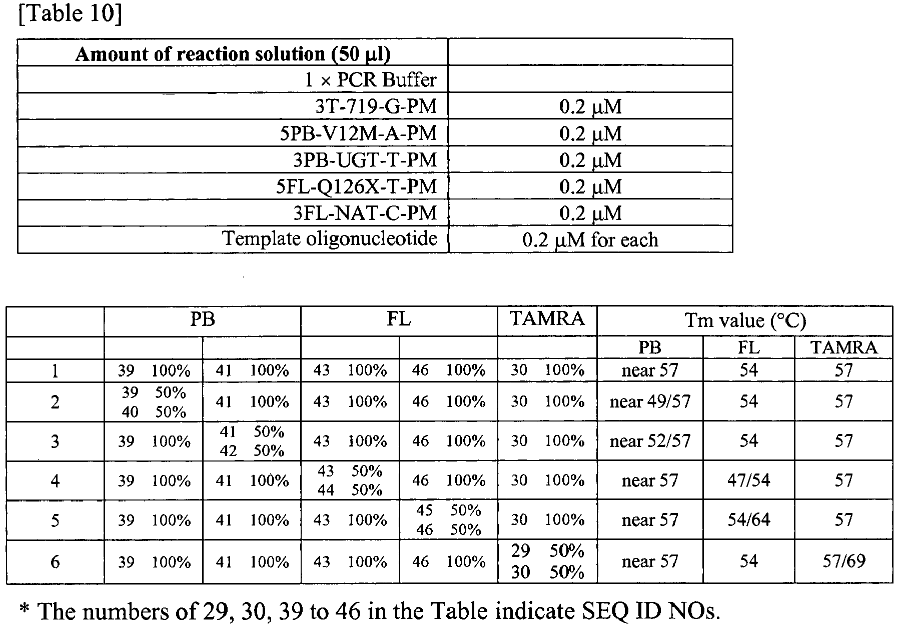

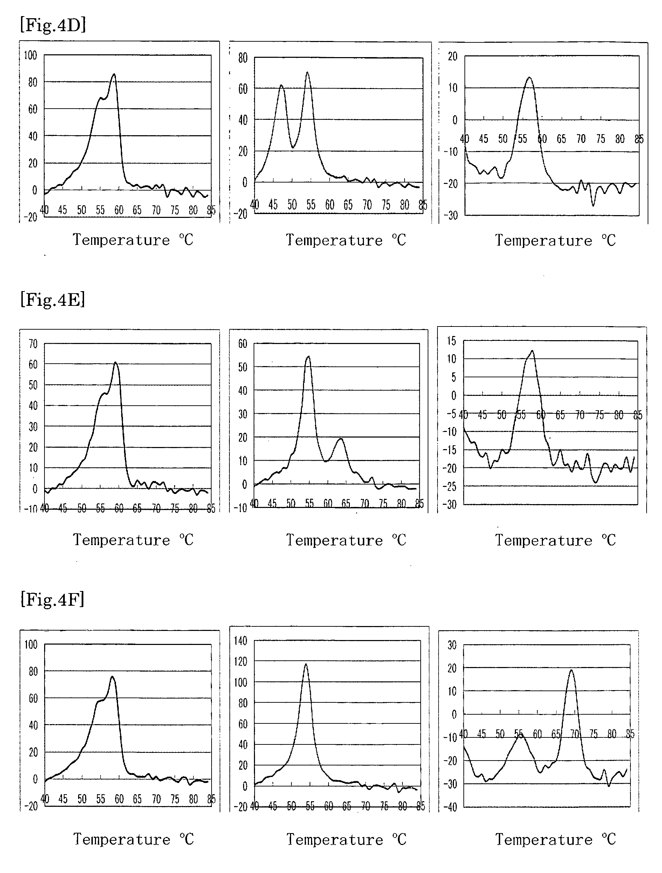

In the results of the Tm analysis performed using the probes of Table 9, regarding Pacific Blue, new peaks were detected only when the samples contained the oligonucleotides of SEQ ID NOs: 40 and 42 (left-hand side charts of Figs. 4B and 4C) and the samples that did not contain the oligonucleotides of SEQ ID NOs: 40 and 42 had only two peaks (left-hand side charts of Figs. 4A, 4D, 4E, 4F).

Regarding BODIPY FL, new peaks were detected only when the samples contained the oligonucleotides of SEQ ID NOs: 44 and 45 (center charts of Figs. 4D and 4E), and regarding the samples without the oligonucleotides of SEQ ID NOs: 44 and 45, only one peak was observed (center charts of Figs. 4A, 4B, 4C, and 4F).

Regarding TAMRA, a new peak was detected only when having the oligonucleotide of SEQ ID NO: 29 (right-hand side chart of Fig. 4F), and regarding the samples without the oligonucleotide of SEQ ID NO: 29, only one peak was observed (right-hand side charts of Figs. 4A to 4E).

From the above, using two kinds of probes: 5PB-V12M-A-PM and 3PB-UGT-T-PM, the two kinds of oligonucleotides (SEQ ID NOs: 40 and 42) were able to be detected at the single detection wavelength of 445 to 480 nm. Additionally, using the two probes: 5FL-Q126X-T-PM and 3FL-NAT-C-PM, mutations of the two kinds of oligonucleotides (SEQ ID NOs: 44 and 45) were able to be detected at the single detection wavelength of 520 to 555 nm. Then, by combining with 3T-719-G-PM, all five kinds of mutations were able to be detected.

### INDUSTRIAL APPLICABILITY

The present invention provides a method that can detect a plurality of genetic mutations associated with medicinal efficacy and/or physical predisposition at one time.

## Claims

1. A method for simultaneously detecting a plurality of nucleotide polymorphisms by melting curve analysis, comprising detecting the plurality of nucleotide polymorphisms at a single wavelength by using a plurality of oligonucleotides each labeled with a fluorescent dye, each of which oligonucleotide hybridizes to a region of a target sequence containing at least one of the plurality of nucleotide polymorphisms, wherein
the detection wavelength of the fluorescent dyes labeling the plurality of oligonucleotides is the same or similar ;
the plurality of oligonucleotides are designed such that the difference between the Tm values of the plurality of oligonucleotides for each of the sequences of said regions to be hybridized containing a first genotype of the nucleotide polymorphisms is 0 to 2°C; and
each of the plurality of oligonucleotides is designed such that the Tm value for the sequence of the region to be hybridized containing the first genotype is different from the Tm value for the sequence of said region to be hybridized containing a second genotype by 3°C or more.

2. The method according to claim 1, wherein the difference between the Tm values of the plurality of oligonucleotides for each of the sequences of said regions to be hybridized containing the first genotype is 0 to 1 °C.

3. The method according to claim 1 or 2 further comprising the simultaneous or sequential performance of a nucleotide polymorphism detection method using one or more fluorescent dye detection wavelengths other than and not similar to the wavelength to be used in the nucleotide polymorphism detection method according to claim 1 or 2.

4. The method according to claim 3 wherein said further nucleotide polymorphism detection method uses two or more fluorescent dye detection wavelengths other than and not similar to the wavelength to be used in the nucleotide polymorphism detection method according to claim 1 or 2.

5. The method according to any one of claims 1 to 4, wherein the first genotype-containing sequence is a wild-type sequence and the second genotype-containing sequence is a sequence having a substitution mutation, an insertion mutation, and/or a deletion mutation of one or more nucleotides in the wild-type sequence.

6. The method according to any one of claims 1 to 5, wherein the oligonucleotides are nucleotide polymorphism detection probes in which the oligonucleotides emit fluorescence when not hybridized to a target sequence, and fluorescence intensity decreases or increases when the oligonucleotides are hybridized to the target sequence.

7. The method according to claim 6, wherein the oligonucleotides are nucleotide polymorphism detection probes in which the fluorescence intensity decreases when the oligonucleotides are hybridized to the target sequence.

8. A kit for simultaneously detecting in solution a plurality of nucleotide polymorphisms at a single wavelength, the kit comprising nucleotide polymorphism detection probes comprising a plurality of oligonucleotides each labeled with a fluorescent dye, each of which oligonucleotide hybridizes to a region of a target sequence containing at least one of the plurality of nucleotide polymorphisms, wherein
the detection wavelength of the fluorescent dyes labeling the plurality of oligonucleotides is the same or similar,
the plurality of oligonucleotides are designed such that the difference beween the Tm values of the plurality of oligonucleotides for each of the sequences of said regions to be hybridized containing a first genotype of the nucleotide polymorphisms is 0 to 2°C; and
each of the plurality of oligonucleotides is designed such that the Tm value for the sequence of the region to be hybridized containing the first genotype is different from the Tm value for the sequence of said region to be hybridized containing a second genotype by 3°C or more.

9. Use of the kit of claim 8 for detecting a plurality of nucleotide polymorphisms at a single wavelength.

10. A method for predicting medicinal efficacy and/or a physical predisposition in an individual, comprising measuring the presence or absence of a nucleotide polymorphism(s) associated with the medicinal efficacy and/or the physical predisposition, by detecting the plurality of nucleotide polymorphisms by the method according to any one of claims 1 to 7.

11. The method according to claim 10 wherein the detection of an EGFR exon 20 insertion polymorphism in a nucleic acid sample obtained from said individual is used to determine the efficacy of gefitinib as an anticancer drug in that individual.

12. A nucleotide polymorphism detection apparatus comprising:
a reaction section that contains a plurality of oligonucleotides each labeled with a fluorescent dye, each of which oligonucleotide hybridizes to a region of a target sequence containing at least one of the plurality ofnucleotide polymorphisms;
a liquid sending section that feeds a sample and/or a reaction solution to the reaction section;
a light source section that emits light for exciting fluorescence in the reaction section;
a control section that controls the temperature of the reaction section; and
a detection section that detects the fluorescence, wherein
the detection wavelength of the fluorescent dyes labeling the plurality of oligonucleotides are the same or similar,
the plurality of oligonucleotides are designed such that the difference between the Tm values of the plurality of oligonucleotides for each of the sequences of said regions to be hybridized containing a first genotype of the nucleotide polymorphisms is 0 to 2°C; and
each of the plurality of oligonucleotides is designed such that the Tm value for the sequence of the region to be hybridized containing the first genotype is different from the Tm value for the sequence of said region to be hybridized containing a second genotype by 3°C or more.

13. A nucleotide polymorphism determination system comprising:
a reaction system that contains a plurality of oligonucleotides each labeled with a fluorescent dye, each of which oligonucleotide hybridizes to a region of a target sequence containing at least one of the plurality of nucleotide polymorphisms;
a liquid sending system that feeds a sample and/or a reaction solution to the reaction system;
a light source system that emits light for exciting fluorescence in the reaction system;
a control section that controls the temperature of the reaction system;
a detection system that detects the fluorescence; and
a determination system that determines the nucleotide polymorphism based on a detection result, wherein
the detection wavelength of the fluorescent dyes labeling the plurality of oligonucleotides are the same or similar,
the plurality of oligonucleotides are designed such that the difference between the Tm values of the plurality of oligonucleotides for each of the sequences of said regions to be hybridized containing a first genotype of the nucleotide polymorphisms is 0 to 2°C; and
each of the plurality of oligonucleotides is designed such that the Tm value for the sequence of the region to be hybridized containing the first genotype is different from the Tm value for the sequence of said region to be hybridized containing a second genotype by 3°C or more.

## Patentansprüche

1. Verfahren zum gleichzeitigen Nachweisen mehrerer Nukleotidpolymorphismen durch Schmelzkurvenanalyse, welches das Nachweisen der mehreren Nukleotidpolymorphismen bei einer einzelnen Wellenlänge durch Verwendung mehrerer Oligonukleotide, die jeweils mit einem fluoreszierenden Farbstoff markiert sind, wobei jedes dieser Oligonukleotide an eine Region einer Zielsequenz hybridisiert, die mindestens einen der mehreren Nukleotidpolymorphismen enthält, umfasst, wobei
die Nachweiswellenlänge der fluoreszierenden Farbstoffe, welche die mehreren Oligonukleotide markieren, die gleiche oder ähnlich ist;
die mehreren Oligonukleotide derart ausgelegt sind, dass der Unterschied zwischen den Tm-Werten der mehreren Oligonukleotide für jede der Sequenzen der zu hybridisierenden Regionen, die einen ersten Genotyp der Nukleotidpolymorphismen enthalten, 0 bis 2 °C beträgt; und
jedes der mehreren Oligonukleotide derart ausgelegt ist, dass sich der Tm-Wert für die Sequenz der zu hybridisierenden Region, die den ersten Genotyp enthält, vom Tm-Wert für die Sequenz der zu hybridisierenden Region, die einen zweiten Genotyp enthält, um 3 °C oder mehr unterscheidet.

2. Verfahren nach Anspruch 1, wobei der Unterschied zwischen den Tm-Werten der mehreren Oligonukleotide für jede der Sequenzen der zu hybridisierenden Regionen, die den ersten Genotyp enthalten, 0 bis 1 °C beträgt.

3. Verfahren nach Anspruch 1 oder 2, welches ferner das gleichzeitige oder sequentielle Durchführen eines Nukleotidpolymorphismus-Nachweisverfahrens unter Verwendung von einer oder mehreren anderen und nicht ähnlichen Nachweiswellenlängen für fluoreszierenden Farbstoff als der Wellenlänge, die bei dem Nukleotidpolymorphismus-Nachweisverfahren nach Anspruch 1 oder 2 zu verwenden ist, umfasst.

4. Verfahren nach Anspruch 3, wobei das weitere Nukleotidpolymorphismus-Nachweisverfahren zwei oder mehr andere und nicht ähnliche Nachweiswellenlängen für fluoreszierenden Farbstoff als die Wellenlänge, die bei dem Nukleotidpolymorphismus-Nachweisverfahren nach Anspruch 1 oder 2 zu verwenden ist, verwendet.

5. Verfahren nach einem der Ansprüche 1 bis 4, wobei die erste Genotyp-enthaltende Sequenz eine Wildtypsequenz ist und die zweite Genotyp-enthaltende Sequenz eine Sequenz ist, die eine Substitutionsmutation, eine Insertionsmutation und/oder eine Deletionsmutation von einem oder mehreren Nukleotiden in der Wildtypsequenz aufweist.

6. Verfahren nach einem der Ansprüche 1 bis 5, wobei die Oligonukleotide Nukleotidpolymorphismus-Nachweissonden sind, bei welchen die Oligonukleotide Fluoreszenz emittieren, wenn sie nicht an eine Zielsequenz hybridisiert sind, und sich die Fluoreszenzintensität verringert oder erhöht, wenn die Oligonukleotide an die Zielsequenz hybridisiert sind.

7. Verfahren nach Anspruch 6, wobei die Oligonukleotide Nukleotidpolymorphismus-Nachweissonden sind, bei welchen sich die Fluoreszenzintensität verringert, wenn die Oligonukleotide an die Zielsequenz hybridisiert sind.

8. Kit zum gleichzeitigen Nachweisen mehrerer Nukleotidpolymorphismen in Lösung bei einer einzelnen Wellenlänge, wobei das Kit Nukleotidpolymorphismus-Nachweissonden umfasst, die mehrere Oligonukleotide umfassen, welche jeweils mit einem fluoreszierenden Farbstoff markiert sind, wobei jedes dieser Oligonukleotide an eine Region einer Zielsequenz hybridisiert, die mindestens einen der mehreren Nukleotidpolymorphismen enthält, wobei
die Nachweiswellenlänge der fluoreszierenden Farbstoffe, welche die mehreren Oligonukleotide markieren, die gleiche oder ähnlich ist;
die mehreren Oligonukleotide derart ausgelegt sind, dass der Unterschied zwischen den Tm-Werten der mehreren Oligonukleotide für jede der Sequenzen der zu hybridisierenden Regionen, die einen ersten Genotyp der Nukleotidpolymorphismen enthalten, 0 bis 2 °C beträgt; und
jedes der mehreren Oligonukleotide derart ausgelegt ist, dass sich der Tm-Wert für die Sequenz der zu hybridisierenden Region, die den ersten Genotyp enthält, vom Tm-Wert für die Sequenz der zu hybridisierenden Region, die einen zweiten Genotyp enthält, um 3 °C oder mehr unterscheidet.

9. Verwendung des Kits nach Anspruch 8 zum Nachweis mehrerer Nukleotidpolymorphismen bei einer einzelnen Wellenlänge.

10. Verfahren zum Vorhersagen medizinischer Effizienz und/oder einer physischen Prädisposition bei einem Individuum, welches das Messen der Gegenwart oder Abwesenheit eines Nukleotidpolymorphismus / von Nukleotidpolymorphismen im Zusammenhang mit der medizinischen Effizienz und/oder der physischen Prädisposition durch den Nachweis der mehreren Nukleotidpolymorphismen durch das Verfahren nach einem der Ansprüche 1 bis 7 umfasst.

11. Verfahren nach Anspruch 10, wobei der Nachweis eines EGFR Exon 20 Insertionspolymorphismus in einer Nukleinsäureprobe erhalten von dem Individuum verwendet wird, um die Effizienz von Gefitinib als ein Antikrebsarzneimittel bei diesem Individuum zu bestimmen.

12. Nukleotidpolymorphismus-Nachweisvorrichtung, welche Folgendes umfasst:
einen Reaktionsabschnitt, der mehrere Oligonukleotide enthält, die jeweils mit einem fluoreszierenden Farbstoff markiert sind, wobei jedes dieser Oligonukleotide an eine Region einer Zielsequenz hybridisiert, die mindestens einen der mehreren Nukleotidpolymorphismen enthält;
einen Flüssigkeitssendeabschnitt, der eine Probe und/oder eine Reaktionslösung zu dem Reaktionsabschnitt zuführt;
einen Lichtquellenabschnitt, der Licht zum Anregen von Fluoreszenz in dem Reaktionsabschnitt emittiert;
einen Steuerabschnitt, der die Temperatur des Reaktionsabschnittes steuert; und
einen Nachweisabschnitt, der die Fluoreszenz erkennt, wobei
die Nachweiswellenlänge der fluoreszierenden Farbstoffe, welche die mehreren Oligonukleotide markieren, die gleiche oder ähnlich ist;
die mehreren Oligonukleotide derart ausgelegt sind, dass der Unterschied zwischen den Tm-Werten der mehreren Oligonukleotide für jede der Sequenzen der zu hybridisierenden Regionen, die einen ersten Genotyp der Nukleotidpolymorphismen enthalten, 0 bis 2 °C beträgt; und
jedes der mehreren Oligonukleotide derart ausgelegt ist, dass sich der Tm-Wert für die Sequenz der zu hybridisierenden Region, die den ersten Genotyp enthält, vom Tm-Wert für die Sequenz der zu hybridisierenden Region, die einen zweiten Genotyp enthält, um 3 °C oder mehr unterscheidet.

13. Nukleotidpolymorphismus-Bestimmungssystem, welches Folgendes umfasst:
ein Reaktionssystem, das mehrere Oligonukleotide enthält, die jeweils mit einem fluoreszierenden Farbstoff markiert sind, wobei jedes dieser Oligonukleotide an eine Region einer Zielsequenz hybridisiert, die mindestens einen der mehreren Nukleotidpolymorphismen enthält;
ein Flüssigkeitssendesystem, das eine Probe und/oder eine Reaktionslösung zu dem Reaktionssystem zuführt;
ein Lichtquellensystem, das Licht zum Anregen von Fluoreszenz in dem Reaktionssystem emittiert;
ein Steuersystem, das die Temperatur des Reaktionssystems steuert; und
ein Nachweissystem, das die Fluoreszenz erkennt, und
ein Bestimmungssystem, das den Nukleotidpolymorphismus basierend auf einem Nachweisergebnis bestimmt, wobei
die Nachweiswellenlänge der fluoreszierenden Farbstoffe, welche die mehreren Oligonukleotide markieren, die gleiche oder ähnlich ist;
die mehreren Oligonukleotide derart ausgelegt sind, dass der Unterschied zwischen den Tm-Werten der mehreren Oligonukleotide für jede der Sequenzen der zu hybridisierenden Regionen, die einen ersten Genotyp der Nukleotidpolymorphismen enthalten, 0 bis 2 °C beträgt; und
jedes der mehreren Oligonukleotide derart ausgelegt ist, dass sich der Tm-Wert für die Sequenz der zu hybridisierenden Region, die den ersten Genotyp enthält, vom Tm-Wert für die Sequenz der zu hybridisierenden Region, die einen zweiten Genotyp enthält, um 3 °C oder mehr unterscheidet.

## Revendications

1. Méthode permettant de détecter simultanément une pluralité de polymorphismes nucléotidiques par une analyse de courbe de fusion, comprenant la détection de la pluralité de polymorphismes nucléotidiques à une longueur d'onde unique au moyen d'une pluralité d'oligonucléotides qui sont chacun marqués par un pigment fluorescent et qui s'hybrident chacun à une région d'une séquence cible contenant au moins un des polymorphismes nucléotidiques de la pluralité de polymorphismes nucléotidiques, où
les longueurs d'onde de détection des pigments fluorescents marquant la pluralité d'oligonucléotides sont identiques ou similaires ;
la pluralité d'oligonucléotides est conçue de sorte que la différence entre les valeurs de Tm de la pluralité d'oligonucléotides correspondant à chacune des séquences desdites régions à hybrider contenant un premier génotype des polymorphismes nucléotidiques soit de 0 à 2 °C ; et
chacun des oligonucléotides de la pluralité d'oligonucléotides est conçu de sorte que la valeur de Tm pour la séquence de la région à hybrider contenant le premier génotype diffère de 3 °C ou plus de la valeur de Tm pour la séquence de ladite région à hybrider contenant un second génotype.

2. Méthode selon la revendication 1, où la différence entre les valeurs de Tm de la pluralité d'oligonucléotides correspondant à chacune des séquences desdites régions à hybrider contenant le premier génotype est de 0 à 1 °C.

3. Méthode selon la revendication 1 ou 2, qui comprend également la réalisation simultanée ou séquentielle d'une méthode de détection d'un polymorphisme nucléotidique utilisant une ou plusieurs longueurs d'onde détection de pigments fluorescents qui sont autres que et non similaires à la longueur d'onde à utiliser dans la méthode de détection de polymorphismes nucléotidiques selon la revendication 1 ou 2.

4. Méthode selon la revendication 3, où ladite méthode additionnelle de détection de polymorphismes nucléotidiques utilise au moins deux longueurs d'onde détection de pigments fluorescents qui sont autres que et non similaires à la longueur d'onde à utiliser dans la méthode de détection de polymorphismes nucléotidiques selon la revendication 1 ou 2.

5. Méthode selon l'une quelconque des revendications 1 à 4, où la séquence contenant le premier génotype est une séquence de type sauvage et la séquence contenant le second génotype est une séquence qui présente une mutation par substitution, une mutation par insertion et/ou une mutation par délétion d'un ou plusieurs nucléotides dans la séquence de type sauvage.

6. Méthode selon l'une quelconque des revendications 1 à 5, où les oligonucléotides sont des sondes de détection de polymorphismes nucléotidiques dans lesquelles les oligonucléotides émettent une fluorescence quand ils ne sont pas hybridés à une séquence cible, et dans lesquelles l'intensité de la fluorescence augmente ou diminue quand les oligonucléotides sont hybridés à la séquence cible.

7. Méthode selon la revendication 6, où les oligonucléotides sont des sondes de détection de polymorphismes nucléotidiques dans lesquelles l'intensité de la fluorescence diminue quand les oligonucléotides sont hybridés à la séquence cible.

8. Nécessaire permettant de détecter, simultanément et en solution, une pluralité de polymorphismes nucléotidiques à une longueur d'onde unique, le nécessaire comprenant des sondes de détection de polymorphismes nucléotidiques comprenant une pluralité d'oligonucléotides qui sont chacun marqués par un pigment fluorescent et qui s'hybrident chacun à une région d'une séquence cible contenant au moins un des polymorphismes nucléotidiques de la pluralité de polymorphismes nucléotidiques, où
les longueurs d'onde de détection des pigments fluorescents marquant la pluralité d'oligonucléotides sont identiques ou similaires ;
la pluralité d'oligonucléotides est conçue de sorte que la différence entre les valeurs de Tm de la pluralité d'oligonucléotides correspondant à chacune des séquences desdites régions à hybrider contenant un premier génotype des polymorphismes nucléotidiques soit de 0 à 2 °C ; et
chacun des oligonucléotides de la pluralité d'oligonucléotides est conçu de sorte que la valeur de Tm pour la séquence de la région à hybrider contenant le premier génotype diffère de 3 °C ou plus de la valeur de Tm pour la séquence de ladite région à hybrider contenant un second génotype.

9. Utilisation du nécessaire de la revendication 8 pour détecter une pluralité de polymorphismes nucléotidiques à une longueur d'onde unique.

10. Méthode permettant de prédire l'efficacité d'un médicament et/ou une prédisposition physique chez un individu, qui comprend la détermination de la présence ou de l'absence d'un ou plusieurs polymorphismes nucléotidiques associés à l'efficacité d'un médicament et/ou la prédisposition physique par une détection de la pluralité de polymorphismes nucléotidiques par la méthode selon l'une quelconque des revendications 1 à 7.

11. Méthode selon la revendication 10, où la détection d'un polymorphisme d'insertion dans l'exon 20 du gène de l'EGFR dans un échantillon d'acide nucléique obtenu chez ledit individu est utilisée pour déterminer l'efficacité du géfitinib en tant que médicament anticancéreux chez cet individu.

12. Dispositif de détection de polymorphismes nucléotidiques qui comprend :
une zone de réaction contenant une pluralité d'oligonucléotides qui sont chacun marqués par un pigment fluorescent et qui s'hybrident chacun à une région d'une séquence cible contenant au moins un des polymorphismes nucléotidiques de la pluralité de polymorphismes nucléotidiques ;
une zone d'alimentation en liquide qui achemine un échantillon et/ou une solution de réaction dans la zone de réaction ;
une zone de source lumineuse qui émet une lumière pour exciter la fluorescence dans la zone de réaction ;
une zone de contrôle qui régule la température de la zone de réaction ; et
une zone de détection qui détecte la fluorescence, où
les longueurs d'onde de détection des pigments fluorescents marquant la pluralité d'oligonucléotides sont identiques ou similaires ;
la pluralité d'oligonucléotides est conçue de sorte que la différence entre les valeurs de Tm de la pluralité d'oligonucléotides correspondant à chacune des séquences desdites régions à hybrider contenant un premier génotype des polymorphismes nucléotidiques soit de 0 à 2 °C ; et
chacun des oligonucléotides de la pluralité d'oligonucléotides est conçu de sorte que la valeur de Tm pour la séquence de la région à hybrider contenant le premier génotype diffère de 3 °C ou plus de la valeur de Tm pour la séquence de ladite région à hybrider contenant un second génotype.

13. Système de détermination de polymorphismes nucléotidiques qui comprend :
un système de réaction contenant une pluralité d'oligonucléotides qui sont chacun marqués par un pigment fluorescent et qui s'hybrident chacun à une région d'une séquence cible contenant au moins un des polymorphismes nucléotidiques de la pluralité de polymorphismes nucléotidiques ;
un système d'alimentation en liquide qui achemine un échantillon et/ou une solution de réaction dans le système de réaction ;
un système de source lumineuse qui émet une lumière pour exciter la fluorescence dans le système de réaction ;
une zone de contrôle qui régule la température du système de réaction ;
un système de détection qui détecte la fluorescence ; et
un système de détermination qui détermine le polymorphisme nucléotidique sur la base du résultat d'une détection, où
les longueurs d'onde de détection des pigments fluorescents marquant la pluralité d'oligonucléotides sont identiques ou similaires ;
la pluralité d'oligonucléotides est conçue de sorte que la différence entre les valeurs de Tm de la pluralité d'oligonucléotides correspondant à chacune des séquences desdites régions à hybrider contenant un premier génotype des polymorphismes nucléotidiques soit de 0 à 2 °C ; et
chacun des oligonucléotides de la pluralité d'oligonucléotides est conçu de sorte que la valeur de Tm pour la séquence de la région à hybrider contenant le premier génotype diffère de 3 °C ou plus de la valeur de Tm pour la séquence de ladite région à hybrider contenant un second génotype.
